# EUROPEAN PATENT SPECIFICATION

(11) **EP 3 570 851 B1**
(45) Date of publication and mention of the grant of the patent: **18.09.2024**
(21) Application number: 18741770.4
(22) Date of filing: 17.01.2018
(51) Int. Cl.: A61K 35/15, A61K 45/06, A61K 9/00, C12N 5/0784, A61F 9/00, A61P 27/02

(54) **ADOPTIVE TRANSFER OF PLASMACYTOID DENDRITIC CELLS TO PREVENT OR TREAT OCULAR DISEASES AND CONDITIONS**
ADOPTIVE ÜBERTRAGUNG VON PLASMACYTOIDEN DENDRITISCHEN ZELLEN ZUR VORBEUGUNG ODER BEHANDLUNG VON AUGENKRANKHEITEN UND LEIDEN
TRANSFERT ADOPTIF DE CELLULES DENDRITIQUES PLASMACYTOÏDES POUR PRÉVENIR OU TRAITER DES MALADIES ET DES ÉTATS OCULAIRES

(30) Priority: 17.01.2017 US 201762447279 P
(43) Date of publication of application: 27.11.2019
(73) Proprietor: Tufts Medical Center, Inc., Boston, MA 02111 (US)
(72) Inventor: HAMRAH, Pedram, Wellesley MA 02482 (US); JAMALI, Arsia, Boston MA 02120 (US)
(74) Representative: Brantsandpatents bv
(86) International application number: PCT/US2018/014095
(87) International publication number: WO 2018/136551

(56) References cited:
- WO-A1-2015/181298
- US-A1- 2011 268 707
- US-A1- 2014 186 309
- US-A1- 2014 301 969
- US-A1- 2014 341 838
- US-A1- 2015 374 791
- US-A1- 2016 361 412
- US-B1- 7 141 607
- PEDRAM HAMRAH: "1R01EY026963-01A1 - THE ROLE OF PLASMACYTOID DENDRITIC CELLS IN OCULAR ANGIOGENESIS", 1 December 2017 (2017-12-01), pages 1, XP055740916, Retrieved from the Internet <URL:https://projectreporter.nih.gov/project_info_description.cfm?aid=9318784&icde=0> [retrieved on 20201016]
- PEDRAM HAMRAH: "1R01EY026963-01A1 - THE ROLE OF PLASMACYTOID DENDRITIC CELLS IN OCULAR ANGIOGENESIS - Details", 1 December 2017 (2017-12-01), pages 1, XP055740919, Retrieved from the Internet <URL:https://projectreporter.nih.gov/project_info_details.cfm?aid=9318784&icde=0> [retrieved on 20201016]
- ARSIA JAMALI ET AL: "Plasmacytoid Dendritic Cells Maintain Corneal Heme-Angiogenic Privilege Through Secretion of Anti-Angiogenic Molecules | IOVS | ARVO Journals", ARVO 2016, vol. 57, 1 September 2016 (2016-09-01), pages 1 - 3, XP055740931
- JAMALI ARSIA ET AL: "Local Adoptive Transfer of Plasmacytoid Dendritic Cells as a Novel Therapeutic Approach for Corneal Nerve Regeneration", vol. 58, 1 June 2017 (2017-06-01), pages 1 - 2, XP093022820, Retrieved from the Internet <URL:https://iovs.arvojournals.org/article.aspx?articleid=2639149>
- SENDRA VICTOR ET AL: "Plasmacytoid Dendritic Cells Mediate T cell Responses by Direct Interaction in Lymph Nodes during Herpes Simplex Virus-1 Keratitis", ANNUAL MEETING OF THE ASSOCIATION-FOR-RESEARCH-IN-VISION-AND-OPHTHALMOLOGY (ARVO), vol. 57, 1 September 2016 (2016-09-01), pages 1 - 2, XP093022850, Retrieved from the Internet <URL:https://iovs.arvojournals.org/article.aspx?articleid=2559213>
- YEUNG A M ET AL: "Fibrin glue inhibits migration of ocular surface epithelial cells", EYE, vol. 30, no. 10, 1 October 2016 (2016-10-01), GB, pages 1389 - 1394, XP093022855, ISSN: 0950-222X, Retrieved from the Internet <URL:https://www.ncbi.nlm.nih.gov/pmc/articles/PMC5129855/pdf/eye2016127a.pdf> DOI: 10.1038/eye.2016.127
- PARK SUSANNA S ET AL: "Intravitreal Autologous Bone Marrow CD34 Cell Therapy for Ischemic and Degenerative Retinal Disorders: Preliminary Phase 1 Clinical Trial Findings", INVEST OPHTHALMOL VIS SCI, vol. 56, 1 January 2015 (2015-01-01), pages 81 - 89, XP093022865, Retrieved from the Internet <URL:https://www.ncbi.nlm.nih.gov/pmc/articles/PMC4288143/pdf/i1552-5783-56-1-81.pdf> DOI: 10.1167/iovs
- YIN, H.: "Idiopathic Choroidal Neovascularization: Intraocular Inflammatory Cytokines And The Effect Of Intravitreal Ranibizumab Treatment", SCIENTIFIC REPORTS, no. 31880, 25 August 2016 (2016-08-25), pages 1 - 8, XP055505405, Retrieved from the Internet <URL:doi:10.1038/srep31880>

## Description

### Background of the Invention

Angiogenesis (AG) is a precisely regulated process through which new blood vessels are formed from pre-existing vessels. Deviations in the dynamic process of angiogenesis, resulting in increase or attenuation of AG, are associated with pathologic conditions. These conditions include corneal neovascularization (NV), retinopathies, and cancers on one end of the spectrum (pathologically increased AG), and atherosclerosis, myocardial infarction, and limb ischemia on the other end of the spectrum (pathologically decreased AG).

Given that corneal avascularity is a prerequisite for the maintenance of vision, in order to maintain its angiogenic privilege, the cornea is equipped with redundant anti-angiogenic mechanisms including, for example, the secretion of anti-angiogenic molecules/small peptides, such as endostatin, angiostatin, thrombospondin (TSP)-1, and TSP-2 (Ellenberg et al., Prog. Ret. Eye Res. 29(3):208-248, 2010), during homeostasis (Cursiefen, Chem. Immunol. Allergy 97:50-57, 2007; Streilein, Nat. Rev. Immunol. 3(11):879-889, 2003). Nevertheless, corneal angiogenic privilege is not absolute and may succumb to an angiogenic environment during disease, resulting in loss of corneal clarity from corneal NV. Corneal NV is a common sequelae of numerous conditions, such as infections, inflammation, trauma, surgery, autoimmune diseases, limbal stem cell deficiency, neoplasms, and contact lens wear (Azar, Trans. Am. Ophthalmol. Soc. 104:264-302, 2006; Beeb, Semin. Cell Dev. Biol. 19(2):125-133, 2008), with up to 1.4 million cases annually in the United States alone (Lee, Surv. Ophthalmol. 43(3):245-269, 1998). Therefore, corneal NV is second only to cataracts as the leading cause of non-refractive visual impairment worldwide (Lee, Surv. Ophthalmol. 43(3):245-269, 1998; Whitcher et al., Bull. World Health Organ. 79(3):214-221, 2001). Corneal NV is associated with complications, including corneal edema, scarring, lipid deposition, and corneal graft rejection, making it a major cause of blindness worldwide (Qazi et al., J. Genet. 88(4):495-515, 2009), and one of the most common causes of blindness in developing countries (site WHOW. Program for the prevention of blindness and deafness: data available on blindness 2006).

Retinal and subretinal or choroidal vascular diseases constitute the most common causes of moderate to severe vision loss in developed countries (Campochiaro, J. Mol. Med. (Berlin, Germany) 91(3):311-321, 2013). Retinal NV occurs in ischemic retinopathies, such as diabetic retinopathy, retinopathy of prematurity, and retinal vein occlusions. In these conditions, ischemia mainly caused by insufficiency of retinal vasculature leads to up-regulation of transcription factor hypoxia inducible factor (HIF)-1α (Wang et al., Proc. Natl. Acad. Sci. U.S.A. 90(9):4304-4308, 1993; Wang et al., Proc. Natl. Acad. Sci. U.S.A. 92(12):5510-5514, 1995; Semeza, J. Appl. Physiol. 88(4):1474-1480, 2000). HIF-1α then joins constitutively expressed HIF-1β to induce transcription of various hypoxia-related genes (Wang et al., Proc. Natl. Acad. Sci. U.S.A. 90(9):4304-4308, 1993; Wang et al., Proc. Natl. Acad. Sci. U.S.A. 92(12):5510-5514, 1995; Semeza, J. Appl. Physiol. 88(4):1474-1480, 2000). Subretinal and choroidal NV occur in diseases of the outer retina and Bruch's membrane, the most prevalent of which is age-related macular degeneration (Campochiaro, J. Mol. Med. (Berlin, Germany) 91 (3):311-321, 2013). Despite a lack of clear evidence on the relevance of hypoxia in development of subretinal and choroidal NV, stabilization of HIF-1α serves as the precipitating event in subretinal and choroidal NV as well. Stabilization of HIF-1 leads to up-regulation of several hypoxia-regulated gene products, such as vascular endothelial growth factor (VEGF) isoforms, angiopoietin 2, and vascular endothelial-protein tyrosine phosphatase (VE-PTP) (Campochiaro, J. Mol. Med. (Berlin, Germany) 91(3):311-321, 2013). Expression of these pro-angiogenic molecules, derived in part from the glial and Müller cells of the inner retina, leads to NV (Ozaki et al., Invest. Ophthal. Vis. Sci. 40(1):182-189, 1999).

Other diseases and conditions of the eye are characterized by nerve degeneration, damage, or inflammation. These diseases include, for example, dry eye disease, neurotrophic keratitis, herpetic keratitis (caused by, e.g., HSV-1), microbial keratitis, corneal infections, ocular herpes (HSV), herpes zoster (shingles), corneal dystrophies, and diabetes. In addition, trauma to the eye caused by, e.g., contact lens wear, chemical or physical burn, injury, surgery (e.g., corneal transplantation, laser assisted in-situ keratomileusis (LASIK), penetrating keratoplasty (PK), automated lamellar keratoplasty (ALK), photorefractive keratectomy (PRK), radial keratotomy (RK), cataract surgery, and corneal incisions), abuse of topical anesthetics, and topical drug toxicity, can cause nerve degeneration, nerve damage, or inflammation, which can result in visual impairment and pain.

The role of plasmacytoid dendritic cells (pDCs) in heme-angiogenesis is known. Jamali et al. 2016 demonstrated the natural endogenous repopulation of the corneal site with pDCs in cases of corneal NV in a mouse model. The resulting effect of the repopulation was regression of corneal NV. However no therapeutic treatment was disclosed. WO2015181298 discloses the isolation of pDCs and their use for treating damage to the optical nerve and/or to retina, retinal pigment degeneration or retinal ischemia. Nonetheless, it is silent about local administration.

There is a need for approaches to prevent and treat diseases and conditions of the eye that are characterized by neovascularization, nerve degeneration or damage, and inflammation.

### Summary of the Invention

The invention provides a plasmacytoid dendritic cell (pDC) or a composition comprising a plasmacytoid dendritic cell and a pharmaceutically acceptable carrier or diluent for use in preventing or treating a disease or condition of the eye in a subject, wherein said pDC or composition is administered to an eye of the subject by intravitreal or sub-retinal injection or by topical administration using a tissue adhesive. The references to the methods of treatment or preventing or treating a disease or condition of the eye throughout this disclosure including summary, description, and figures, are to be interpreted as references to the plasmacytoid dendritic cell (pDC) or the composition comprising a plasmacytoid dendritic cell for as as defined above.

In various embodiments, the disease or condition is characterized by neovascularization. In some examples, the neovascularization is corneal neovascularization. In these examples, the subject may have or be at risk of developing, for example, corneal infection, inflammation, autoimmune disease, limbal stem cell deficiency, neoplasia, uveitis, keratitis, corneal ulcers, glaucoma, rosacea, lupus, dry eye disease, or ocular damage due to trauma, surgery, or contact lens wear.

In other examples, the neovascularization is retinal neovascularization. In these examples, the subject may have or be at risk of developing ischemic retinopathy, diabetic retinopathy, retinopathy of prematurity, retinal vein occlusion, ocular ischemic syndrome, sickle cell disease, Eales' disease, or macular degeneration.

In yet other examples, the neovascularization is choroidal neovascularization. In these examples, the subject may have or be at risk of developing inflammatory neovascularization with uveitis, macular degeneration, ocular trauma, sickle cell disease, pseudoxanthoma elasticum, angioid streaks, optic disc drusen, myopia, malignant myopic degeneration, or histoplasmosis.

In other embodiments, the disease or condition of the eye is characterized by ocular nerve degeneration or damage, e.g., corneal nerve damage. In various examples, the subject has or is at risk of developing dry eye disease, corneal infection, or corneal neurotrophic keratopathy. In other examples, the subject has or is at risk of experiencing ocular damage due to trauma, surgery, contact lens wear, dry eye disease, herpetic keratitis that is optionally caused by HSV-1, neurotrophic keratitis, corneal infections, excessive or improper contact lens wear, ocular herpes (HSV), herpes zoster (shingles), chemical and physical burns, injury, trauma, surgery (including corneal transplantation, laser assisted in-situ keratomileusis (LASIK), penetrating keratoplasty (PK), automated lamellar keratoplasty (ALK), photorefractive keratectomy (PRK), radial keratotomy (RK), cataract surgery, and corneal incisions), abuse of topical anesthetics, topical drug toxicity, corneal dystrophies, vitamin A deficiency, diabetes, and microbial keratitis.

In other embodiments, the disease or condition of the eye is characterized by inflammation. For example, the disease or condition may be selected from: episcleritis, scleritis, uveitis (e.g., anterior uveitis (including iritis and iridocyclitis), intermediate uveitis (including vitritis and pars planitis), posterior uveitis (including retinitis, choroiditis, chorioretinitis, and neuroretinitis), panuveitis (infectious) (including endophthalmitis), and panuveitis (non-infectious)), and retinal vasculitis

The plasmacytoid dendritic cells can be obtained from the subject to whom they are administered or can be obtained from an individual (e.g., a human) and/or species different from the subject to whom they are administered.

Other features and advantages of the invention will be apparent from the following detailed description, the drawings, and the claims.

### Brief Description of the Drawings

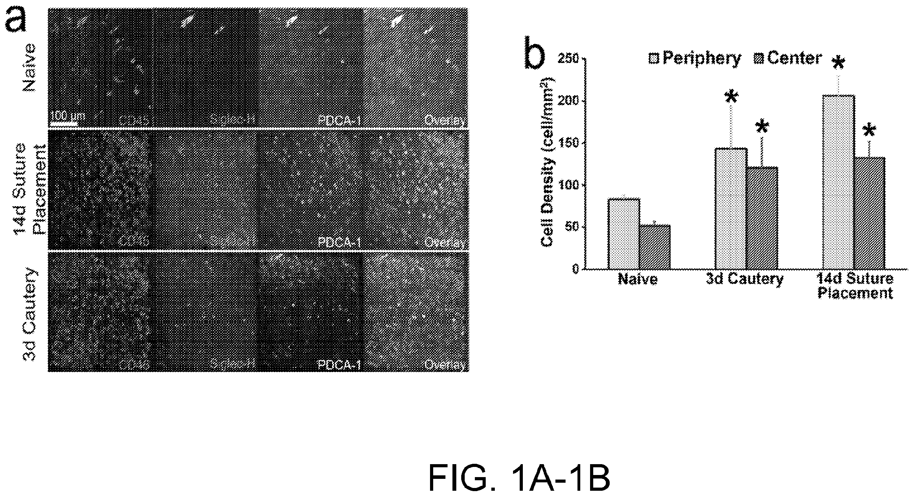

**Figs. 1A and 1B****.** Presence of resident plasmacytoid dendritic cells in naive corneas and substantial increase in their density upon inflammation. Stacked confocal micrograph of whole-mounted cornea stained with CD45 (pan-leukocyte marker), Siglec-H, and PDCA-1 (two specific murine pDC marker) in naive state (Fig. 1A, upper panel), 14 days following suture placement (Fig. 1A, middle panel), and 3 days after thermal cautery (Fig. 1A, lower panel). Quantification of CD45⁺ PDCA-1⁺ cell density in corneas in steady state, and during inflammation (Fig. 1B). Error bars; standard deviation, *P<0.05 compared to naive corneas.

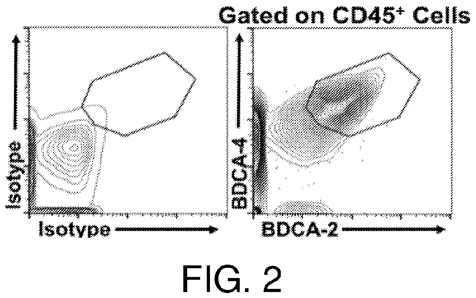

**Fig. 2**. Flow cytometric evaluation of human corneas indicates presence of resident pDCs. FACS analysis of digested human corneas stained with BDCA2, BDCA4 (two specific human pDC markers), CD45, or isotypes shows co-stained CD45⁺ BDCA2⁺ BDCA4⁺ cells, indicating presence of pDCs in the human cornea.

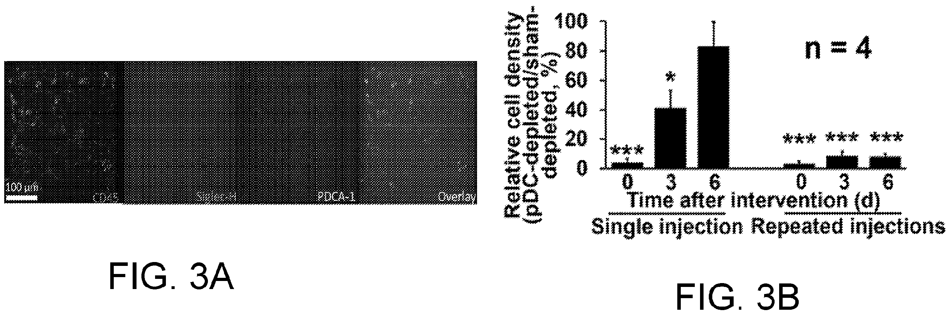

**Figs. 3A and 3B****.** Local depletion of pDCs. Stacked confocal micrograph of a whole-mounted cornea, stained with Siglec-H, PDCA-1 (two specific pDC markers) and CD45 (pan-leukocyte marker) 36 hours after subconjunctival injection of 30 ng Diphtheria toxin (DT) in pDC-DTR mouse indicates successful depletion of pDCs (Fig. 3A). Scale bar; 100µm. Quantification of pDC density in cornea after single versus multiple injections of DT (n =4) (Fig. 3B). Error bars; standard deviation, *, p<0.05; ***, p<0.001.

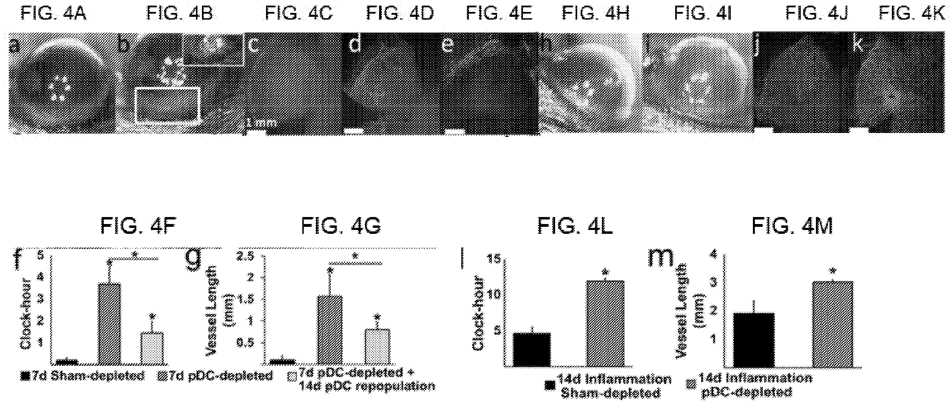

**Figs. 4A-4M****.** pDCs depletion results in increased angiogenesis in steady state and during corneal inflammation. Photograph with surgical microscope showing corneas after 7-days of subconjunctival injections with 30 ng DT in WT mouse (control) (Fig. 4A), pDC-DTR mouse (pDC-depleted state) (inset: 2.5X magnification of the white rectangle) (Fig. 4B), 14 days after suturing in sham-depleted control (Fig. 4H) and pDC-depleted corneas (Fig. 4I). Clinical quantification of NV in pDC-depleted, sham-depleted, and pDC-depleted followed by 14 days of pDC repopulation (Fig. 4F) and 14 days following suturing in sham-depleted versus pDC-depleted corneas (Fig. 4L). Stacked confocal micrograph of whole-mounted cornea stained with CD31 at 7 days sham-depletion (Fig. 4C), pDC-depletion (Fig. 4D), and pDC-depletion followed by 14 days pDC repopulation (Fig. 4E), 14 days after suturing in sham-depleted control (Fig. 4J), and pDC-depleted state (Fig. 4K). Quantification of vessel length of confocal micrographs in sham-depleted, pDC-depleted, and pDC-depleted followed by 14 days of pDC repopulation (Fig. 4G) and 14 days following induction of inflammation in pDC-depleted state vs. sham-depleted controls (Fig. 4M). Error bars; standard deviation, asterisks, p<0.01.

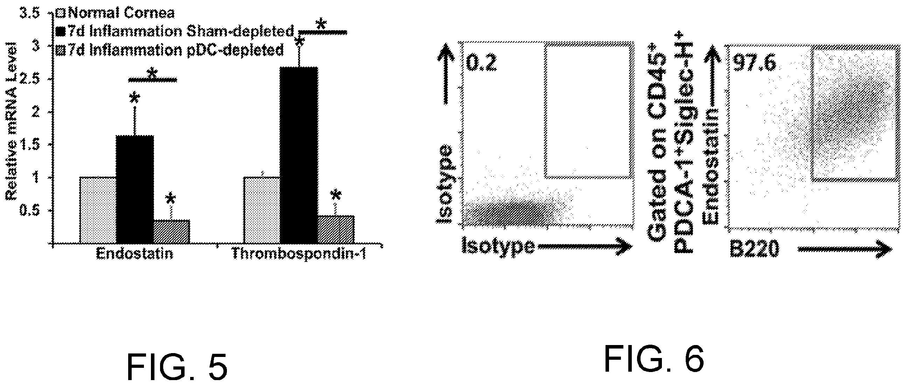

**Fig. 5****.** pDC depletion results in decreased thrombospondin-1 and Endostatin mRNA level. qRT-PCR shows decreased thrombospondin-1 and endostatin mRNA levels 7 days after induction of inflammation in pDC depleted versus sham-depleted control group receiving subconjunctival Diphtheria toxin. Error bars; standard deviation, asterisks, p<0.01.
**Fig. 6****.** Endostatin co-localizes with corneal pDCs. FACS dot plots on 7-day sutured corneas stained with CD45 (pan-leukocyte marker), PDCA-1, CD45R/B220, Siglec-H (pDC markers) and endostatin.

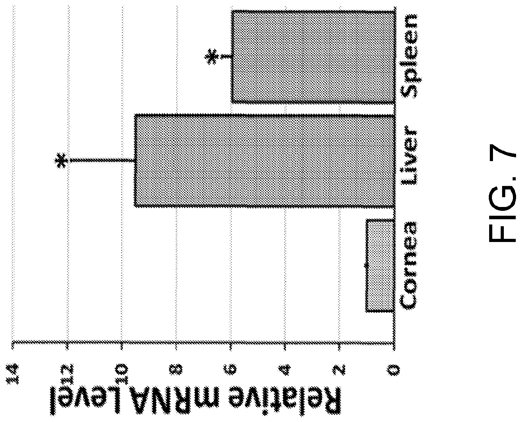

**Fig. 7****.** Higher VEGF-A mRNA levels in the spleen and liver compared to the cornea. qRT-PCR on RNA extracted from naive liver, spleen, and cornea (n=3) shows higher VEGF-A mRNA in vascularized tissues of liver and spleen. Error bars; standard deviation *, p<0.05

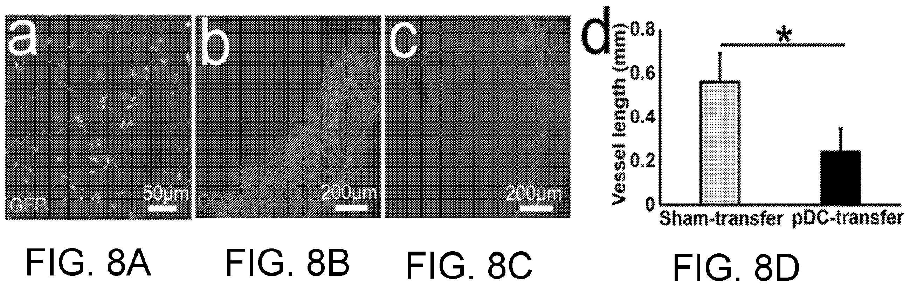

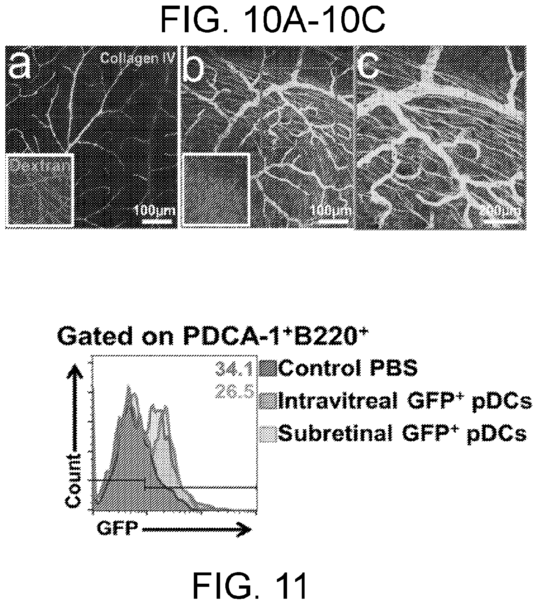

**Figs. 8A-8D****.** Local adoptive transfer of GFP⁺ plasmacytoid dendritic cells to the cornea leads to diminished neovascularization induced by suture placement. Representative whole-mount corneal confocal micrograph of WT B6 mice 48 hours after suture placement and mechanical debridement of central cornea epithelium followed by adoptive transfer of 10⁴ GFP⁺ pDCs using TISSEEL tissue glue shows successful transfer of pDCs to the cornea (Fig. 8A); CD31 stained whole-mounted corneas after 7-day suture placement and control TISSEEL only (Fig. 8B) or pDC transfer (Fig. 8C; n=4/group). Quantification of neovascular vessel length indicates reduced NV in pDC-transferred mice (Fig. 8D). Scale bars 50 µm (Fig. 8A), 200 µm (Fig. 8B, Fig. 10C); asterisk, p <0.01.

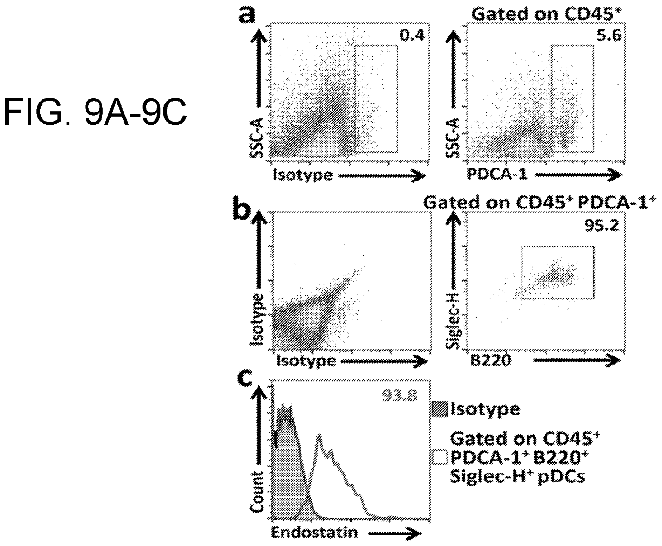

**Figs. 9A-9C****.** Flow cytometric analysis of naive retinas depicts presence of resident pDCs under homeostatic condition. Naive wild type C57BL/6 mice were euthanized (n=5), retinas were excised and digested with collagenase and DNase to yield single cell suspension of retina. Next, cells were stained with primary conjugated antibodies against CD45, PDCA-1, Siglec-H, CD45R/B220, endostatin, or their respective isotype controls. After gating out debris and doublets, events were gated on CD45. Subsequently, events were gated on CD45⁺ PDCA-1⁺ singlets (Fig. 9A) to demonstrate CD45⁺ PDCA-1⁺ Siglec-H⁺ B220⁺ pDCs (Fig. 9B). Further analysis shows that pDCs express endostatin (Fig. 9C).

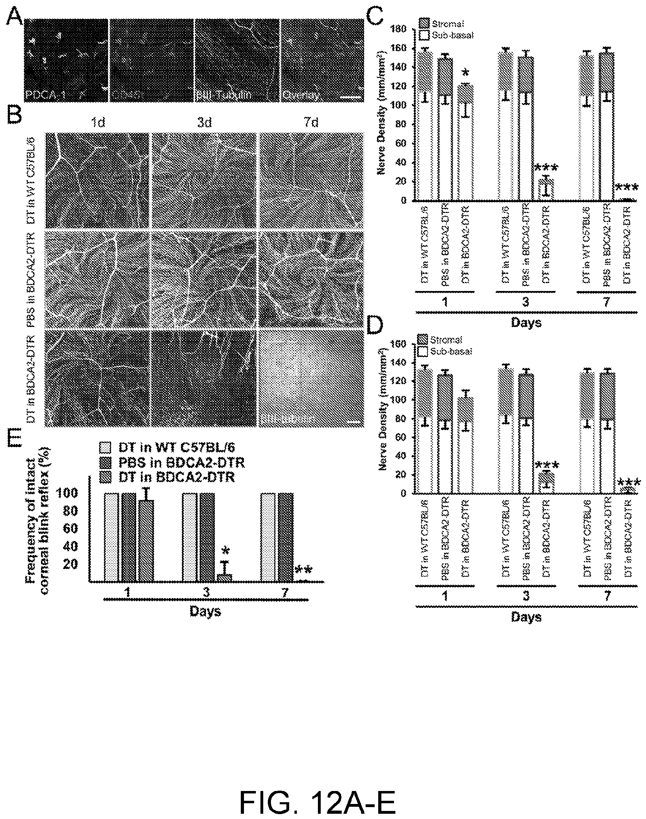

**Figs. 10A-10C****.** Local retinal pDC depletion is accompanied with neovascularization and increased vascular permeability. Representative whole-mount retinal confocal micrograph of control WT B6 (Fig. 10A) and pDC-DTR (Fig. 10B) mice receiving intravitreal 30 ng DT every 48 hours stained with collagen IV. White insets demonstrate dextran vascular leakage in (Fig. 10B) compared to control (Fig. 10A). Red inset is magnified in (Fig. 10C) to show neo-vessels. Scale bars, 100 µm (Fig. 10A, Fig. 12B), 200 µm.
**Fig. 11****.** Adoptive transfer of GFP⁺ pDCs to the naive retina. 2×10⁴ GFP⁺ sorted pDC or PBS were injected intravitreally or subretinally to WT B6 mice. 24 hours later, retinas were subjected to FACS using PDCA-1 and CD45R/B220 antibodies. FACS histogram shows presence of GFP⁺ PDCA-1⁺ CD45R/B220⁺ pDCs among non-GFP (host-derived) pDCs in the retina, indicating feasibility of adoptive transfer of pDCs to retina. The graph is representative of 3 independent experiments.
**Figs. 12A-12E****.** Depletion of plasmacytoid dendritic cells is accompanied by abrupt corneal nerve degeneration and sensory function diminishment. (Fig. 12A) Confocal micrograph of naive WT C56BL/6 corneal whole mount demonstrates spatial proximity of resident pDCs, identified by expression of CD45 (red) and PDCA-1 (green), and corneal nerves (white). Scale bar, 100 µm. (Figs. 12B-12D) Local depletion of corneal pDCs by subconjunctival injection of DT in BDCA2-DTR mice is accompanied by degeneration of sub-basal and stromal nerve plexuses of central (Figs. 12B and 12C) and peripheral cornea (Fig. 12D). Nerve plexuses in control groups, consisting of WT C57BL/6 mice receiving DT and BDCA2-DTR mice treated with PBS, remained intact. (Fig. 12B) Confocal micrograph of the center of whole-mounted corneas stained with βIII-Tubulin (a pan-neuronal marker); (Figs. 12C-12D) Quantification of the corneal nerves density. Scale bar in (Fig. 12B), 100 µm. Error bars show SD, n=3-4 in each group. *, p<0.05; ***, p <0.001. P values are calculated by ANOVA with Bonferroni post hoc. (Fig. 12E) Frequency of intact corneal blink reflex is diminished in the central cornea following pDC depletion versus control groups. Error bars show standard deviation of 3 independent experiments, n=3-5 in each group in each experiment. * p<0.01; ** p<0.001. P values are calculated by Chi square.

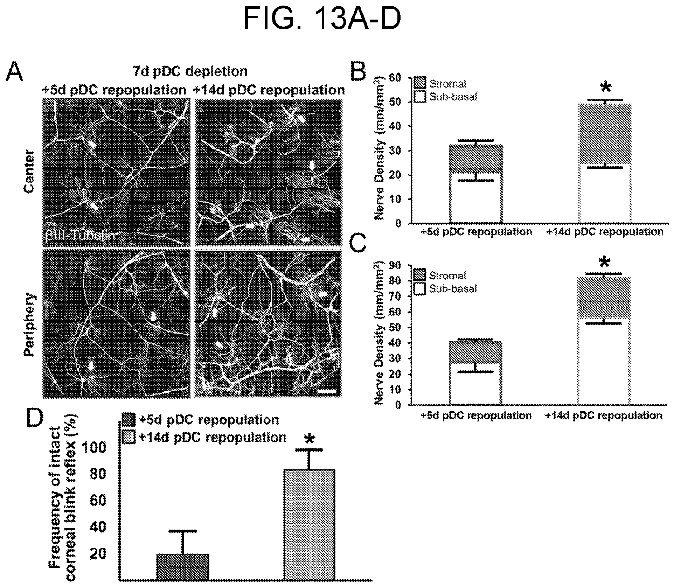

**Figs. 13A-13D****.** Repopulation of plasmacytoid dendritic cells after initial depletion induces nerve regeneration in cornea and re-establishes corneal sensory function. (Fig. 13A) Confocal micrograph of whole-mounted corneas stained with βIII-Tubulin (a pan-neuronal marker) in center (upper panel) and periphery (lower panel) 5 and 14 days following stopping DT injection (pDC repopulation). Scale bar, 100 µm. (Figs. 13B-13C) Quantification of corneal nerve density 5 and 14 following stopping DT injections (pDC repopulation) in center (Fig. 13B) and periphery (Fig. 13C) of cornea. Error bars show SD, n=3-4 in each group. *, p <0.001. P values are calculated by ANOVA with Bonferroni post hoc. (Fig. 13D) Frequency of intact corneal blink reflex in the central cornea following pDC repopulation. Error bars show standard deviation of 3 independent experiments, n=3-5 in each group in each experiment. * p<0.001. P values are calculated by Chi square.

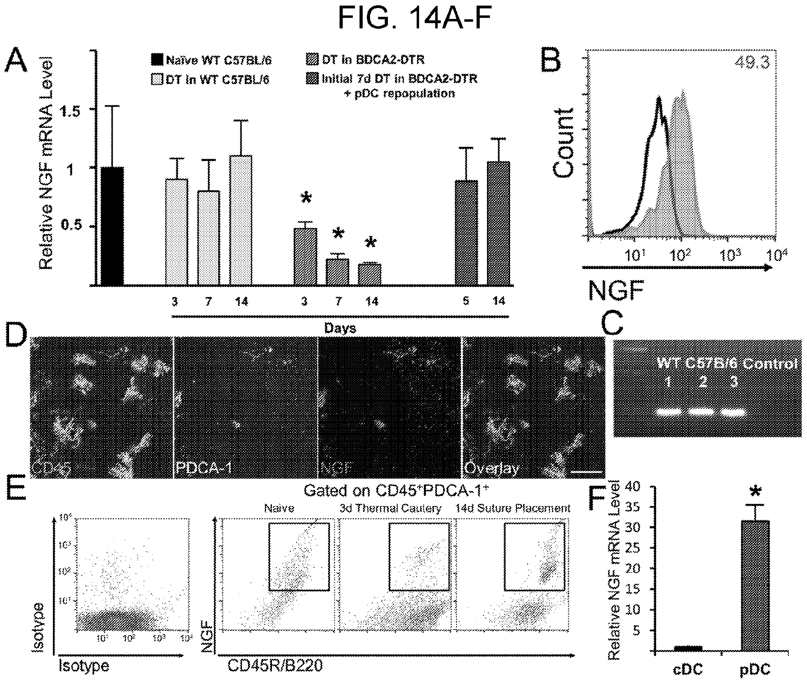

**Figs. 14A-14F****.** Plasmacytoid dendritic cells are vital source of NGF in the cornea. (Fig. 14A) Relative NGF mRNA level in corneal stroma in naive, pDC depleted, and control DT administered WT C57BL/6 mice, as well as upon re-population of pDCs. Bars show SD of 3 independent biological experiments, each on pooled 6-8 corneal stoma per group. P values are calculated by ANOVA with Bonferroni post hoc. (Fig. 14B) Representative FACS analysis of sorted GFP-tagged pDCs from solenocytes of transgenic DPE-GFP×RAG1^{-/-} mice stained for NGF. (Fig. 14C) Agarose gel electrophoresis on PCR product with NGF primer on the cDNA synthetized from the RNA extracted from sorted splenic pDCs from naive DPE-GFP×RAG1^{-/-} mouse (3 different samples) or control lacking template RNA. Image is representative of 3 biologic repeats. (Fig. 14D) Representative confocal micrograph of whole-mount WT C57BL/6 naive cornea stained with CD45 (green; pan-leukocyte marker), PDCA-1 (white; pDC marker), and NGF (red) highlights co-staining of pDCs and NGF in the cornea. Scale bar, 50 µm. (Fig. 14E) Representative FACS analysis of naive, 3d post thermal cautery, and 7d sutured single corneal cells, following removing debris, dead cells, and doublets, and gating on CD45 and PDCA-1 shows co-localization of pDCs (CD45⁺PDCA-1⁺D45R/B220⁺) with NGF. (Fig. 14F) Graph showing relative NGF mRNA levels in cDCs and pDCs. Error bars show standard deviation. * p<0.01. P value is calculated with T test.

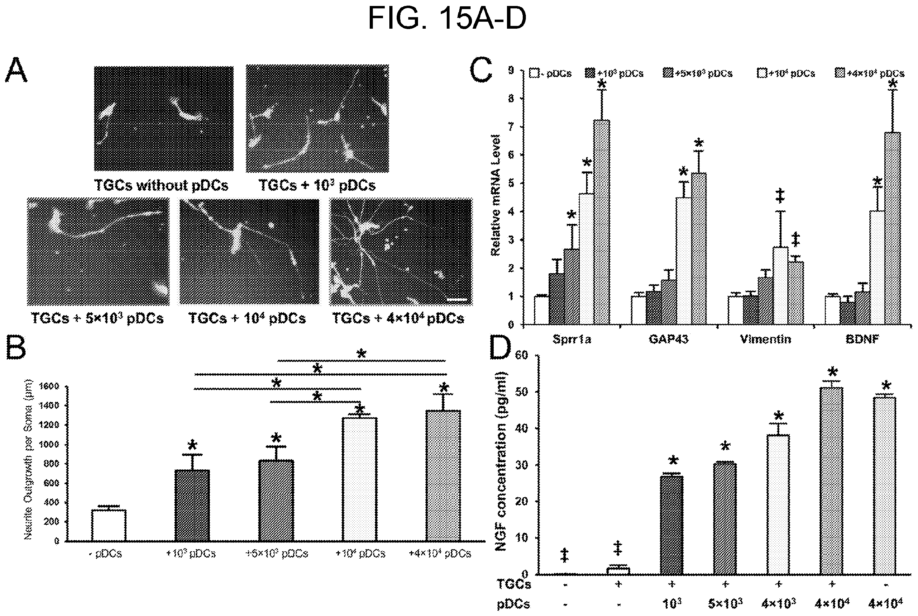

Figs. 15A-15D. Plasmacytoid dendritic cells promote neurite outgrowth in trigeminal ganglion cell culture through secretion of nerve growth factor *in vitro.* (Fig. 15A) Imaged TGCs stained with 1 µM calcein following 3 days co-culture without and with indicated number of pDCs. Scale bar, 50 µm. (Fig. 15B) Quantified neurite outgrowth per soma of TGCs following 3 days co-culture without and with indicated number of pDCs. Bars show SD of 3 independent experiments, each in triplicate. * p<0.001. P values are calculated by ANOVA with Bonferroni post hoc. (Fig. 15C) Relative mRNA levels of Sprr1a, GAP43, Vimentin, and BDNF in TGCs following 3 days co-culture without and with indicated number of pDCs. Bars show SD of 3 independent experiments, each in triplicate. ‡ p<0.05; * p<0.001 . P values are calculated by ANOVA with Bonferroni post hoc. (Fig. 15D) NGF protein level in the cell culture media following 3 days co-culture of TGCs and indicated number of pDCs as well as in TGCs or pDCs monoculture. Error bars show standard deviation of 3 independent experiments. $ less than detection limit. * p<0.001. P values are calculated by ANOVA with Bonferroni post hoc.

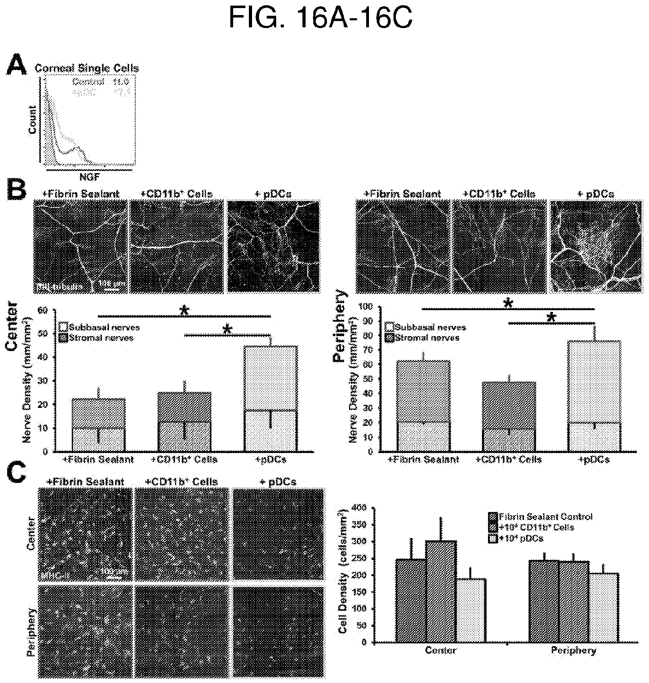

**Figs. 16A-16C****.** Adoptive transfer of plasmacytoid dendritic cells enhances nerve regeneration. (Fig. 16A) Flow cytometry histogram showing increased frequency of NGF-expressing cells in the cornea 3 days following trephination and adoptive transfer of 10⁴ pDCs compared with TISSEEL fibrin sealant control. (Fig. 16B) Confocal micrographs of nerve fibers stained with βIII-tubulin and their densities in the center and periphery of the cornea 14 days after trephination and adoptive transfer of TISSEEL fibrin sealant control, 10⁴ pDCs, or 10⁴ CD11b⁺ myeloid cells. (Fig. 16C) Confocal micrographs and quantification of MHC-II⁺ cells in the center and periphery of the cornea 14 days after trephination and adoptive transfer of TISSEEL fibrin sealant control, 10⁴ pDCs, or 10⁴ CD11b⁺ myeloid cells. Scale bars: 100 µm, Error bars, standard deviation, * p<0.05.

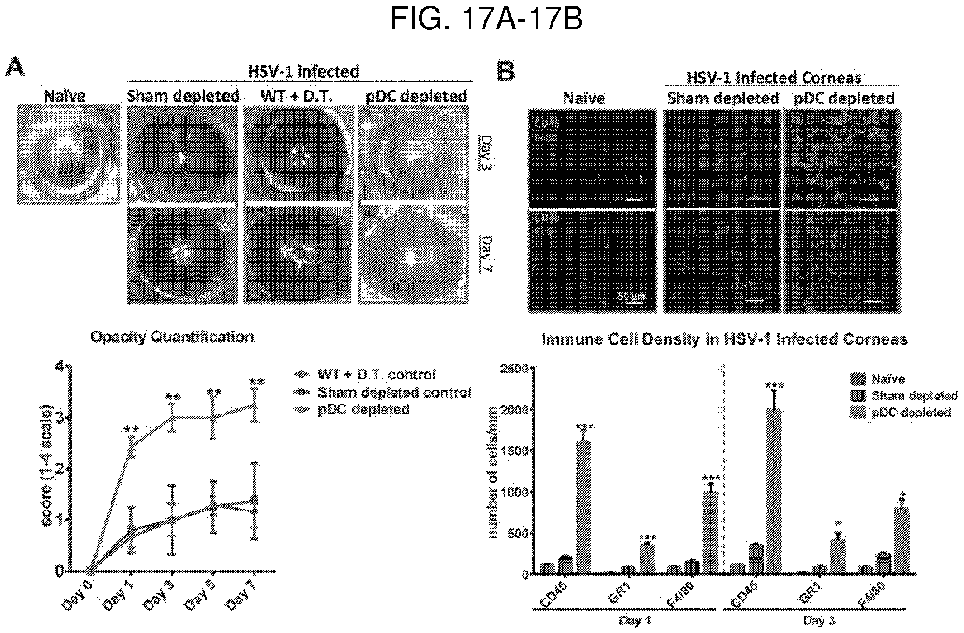

**Figs. 17A and 17B****.** Depletion of plasmacytoid dendritic cells leads to increased severity of acute HSV-1 keratitis. 24 hours following local pDC depletion, corneas were scarified and inoculated with 2×10⁶ PFU HSV-1 McKrae strain. (Fig. 17A) Representative clinical image of corneas on days 3 and 7 following inoculation of HSV-1 and clinical opacity scores in pDC depleted and control mice (C57BL/6 mice receiving subconjunctival DT and pDC-DTR mice treated with subconjunctival PBS, called sham-depleted). (Fig. 17B) Representative confocal micrograph of whole-mounted corneas stained with CD45, Gr-1, and F4/80 on day 3 following HSV-1 inoculation and quantification of the density of data. Error bars, standard error of mean (A) and standard deviation (B), Scale bar: 50 µm, * p<0.05, ** p<0.01, *** p<0.001.

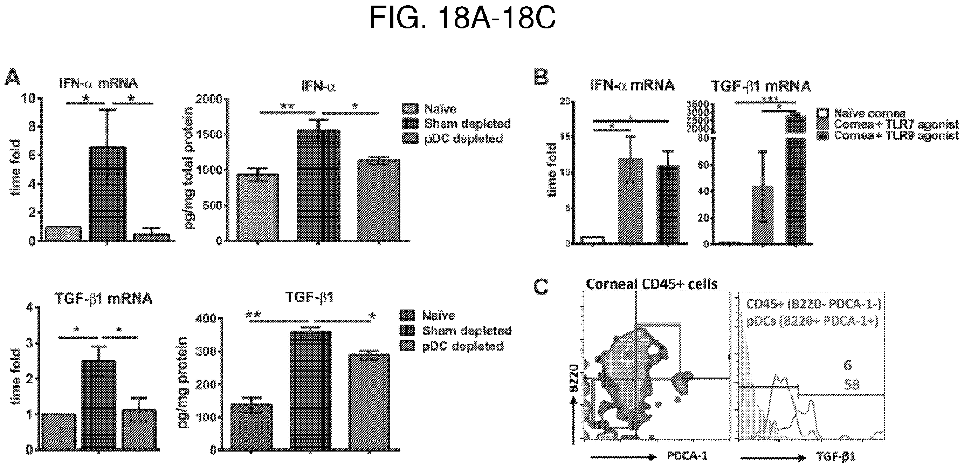

**Figs. 18A-18C****.** Depletion of plasmacytoid dendritic cells is accompanied with decreased IFN-α and TGF-β1 in acute HSV-1 keratitis. (Fig. 18A) mRNA and protein levels of IFN-α and TGF-β1 in the corneal stomas in sham- and pDC-depleted corneas on day 3 following HSV-1 inoculation. (Fig. 18B) IFN-α and TGF-β1 mRNA levels in sorted corneal GFP⁺ pDCs from DPE-GFP×RAG1^{-/-} mice 24 hours after inoculation of 10 µg Imiquimod (TLR7 agonist), 10 µg CpG-ODN (TLR9 agonist), or control ODN. (Fig. 18C) Flow cytometric plots of single cell suspension of normal C57BL/6 corneas stained with CD45, PDCA-1, CD45R/B220, and TGF-β1, representing the frequency of TGF-β1⁺ CD45⁺ PDCA-1⁻CD45R/B220⁻ leukocytes and CD45⁺PDCA-1⁺ CD45R/B220⁺ pDCs. Error bars, standard deviation, * p<0.05, ** p<0.01, *** p<0.001.

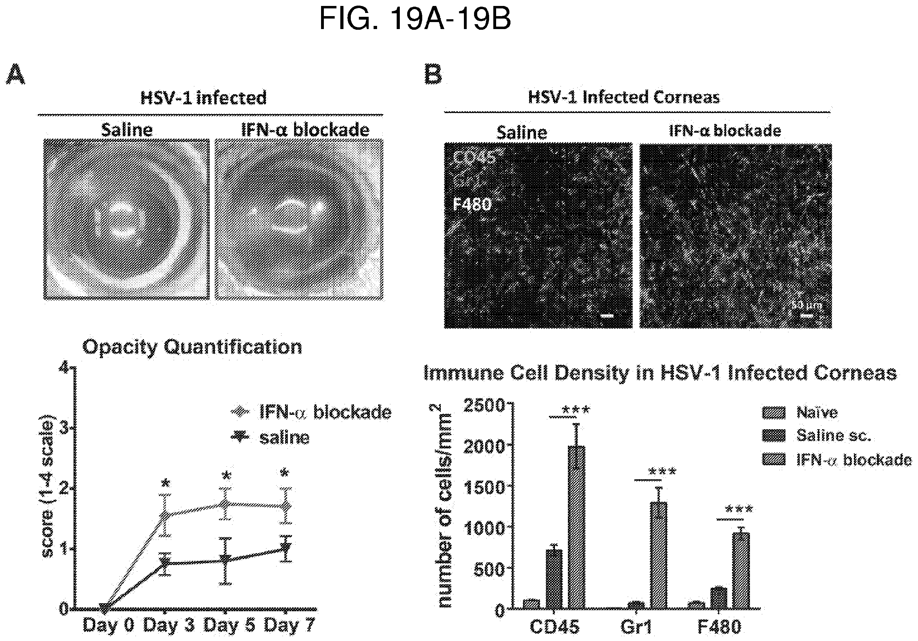

**Figs. 19A and 19B****.** IFN-α blockade enhances the severity of inflammation in acute HSV-1 keratitis. (Fig. 19A) Representative clinical image of corneas on day 3 following inoculation of HSV-1 and relevant clinical opacity scores in C57BL/6 mice receiving normal saline (control) or anti-INF-α antibodies. (Fig. 19B) Representative confocal micrograph of whole-mounted corneas stained with CD45, Gr-1, and F4/80 on day 3 following HSV-1 inoculation and quantification of the density of data. Error bars, standard error of mean (A) and standard deviation (B), Scale bar: 50 µm, * p<0.05, *** p<0.001.

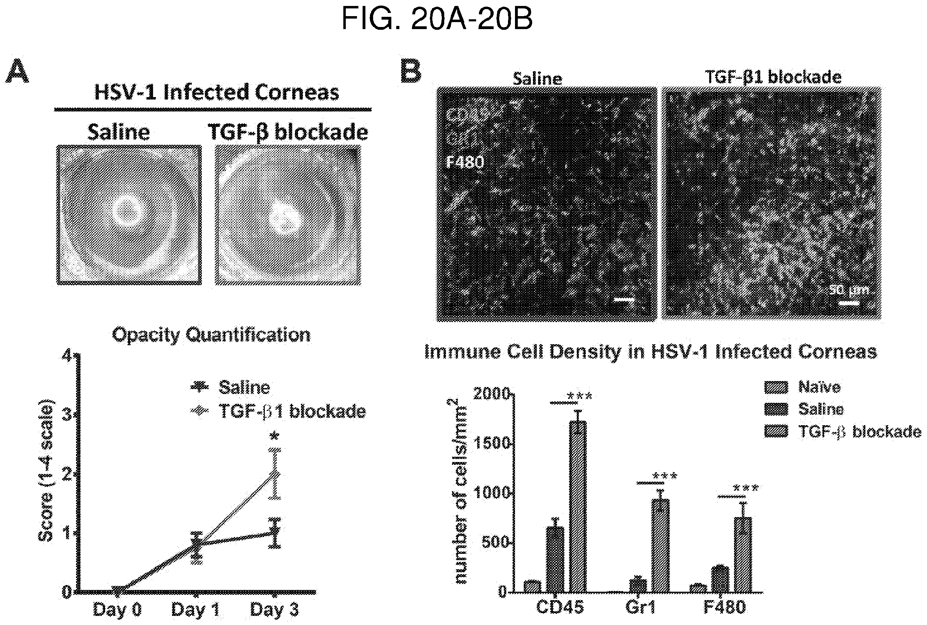

**Figs. 20A and 20B****.** TGF-β1 blockade augments the severity of inflammation in acute HSV-1 keratitis. (Fig. 20A) Representative clinical image of corneas on day 3 following inoculation of HSV-1 and relevant clinical opacity scores in C57BL/6 mice treated with normal saline (control) or anti-TGF-β1 antibodies. (Fig. 20B) Representative confocal micrograph of whole-mounted corneas stained with CD45, Gr-1, and F4/80 on day 3 following HSV-1 inoculation and quantification of the density of data. Error bars, standard error of mean (A) and standard deviation (B), Scale bar: 50 µm, * p<0.05, *** p<0.001.

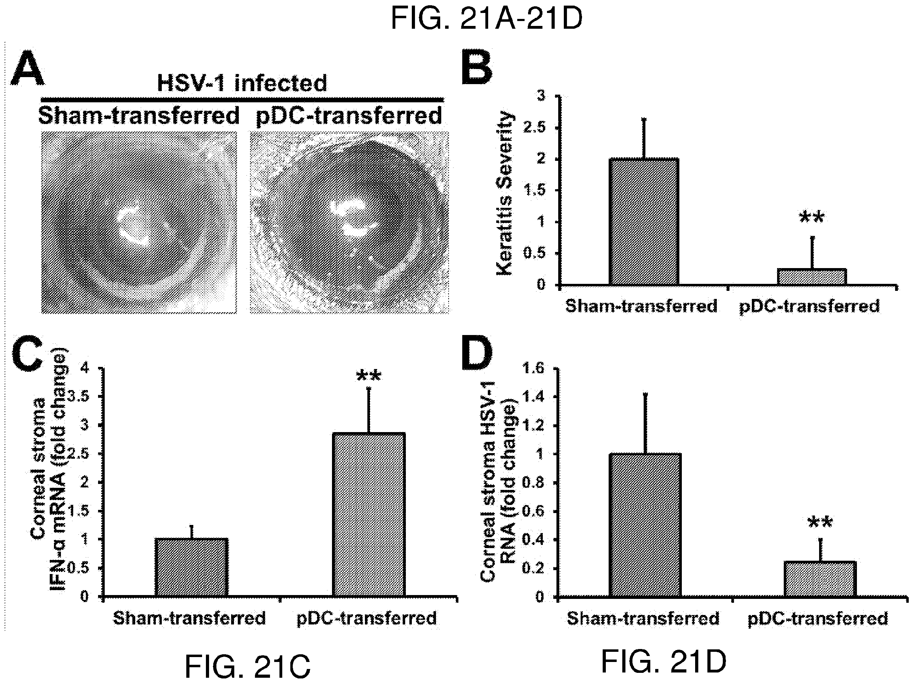

**Figs. 21A-21D****.** Local adoptive transfer of plasmacytoid dendritic cells diminishes clinical severity and promotes viral clearance in acute HSV-1 keratitis. 24 hours following culture, 10⁴ isolated splenic pDCs were resuspended in fibrin sealant and transferred to the center of the cornea WT C57BL/6 mice subsequent to debridement of the epithelium of central cornea. 24 hours later corneas were inoculated with 2×10⁶ PFU HSV-1 McKrae strain. (Fig. 21A) Representative clinical image of corneas on day 5 following inoculation of HSV-1 in sham- and pDC-transferred mice. (Fig. 21B) Quantification of clinical severity of HSV-1 keratitis indicates subsided corneal opacity in mice receiving pDCs in contrast to sham-transferred corneas. qRT-PCR reveals higher IFN-α (Fig. 21C) and lower HSV-1 gB RNA (Fig. 21D) in corneal stroma of mice receiving additional pDCs. Error ars, standard deviation, ** p<0.01 (compared to sham-transferred controls).

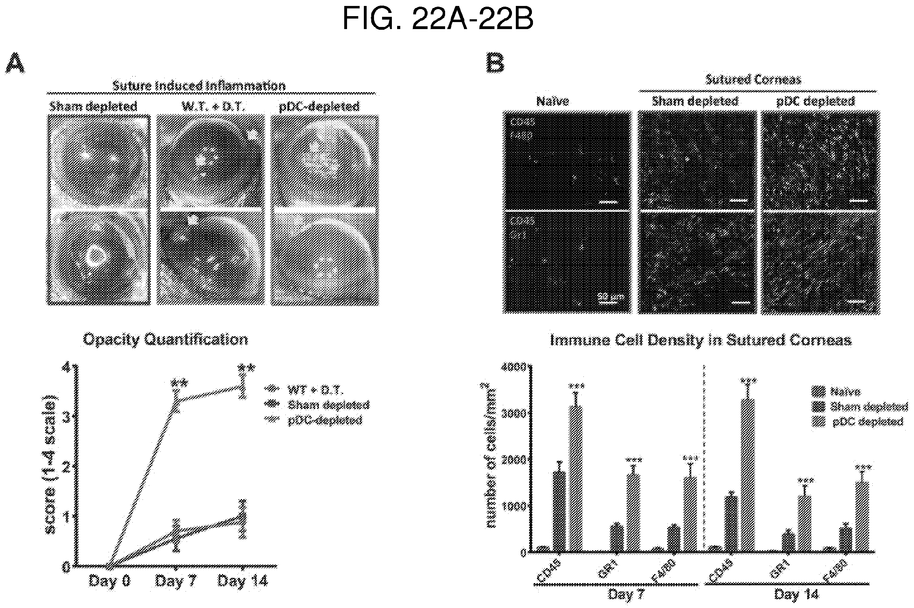

**Figs. 22A and 22B****.** Local depletion of corneal plasmacytoid dendritic cells enhances severity of sterile inflammation. Corneal suture placement was preformed 24 hours after initial subconjunctival injection of DT to pDC-DTR and WT C57BL/6 mice or PBS to pDC-DTR (called sham-control) mice. (Fig. 22A) Representative clinical image of the corneas on day 7 following suture placements (Yellow arrows point to sutures) and quantification of corneal opacity. (Fig. 22B) Representative confocal micrographs of whole-mounted corneas stained with CD45, Gr-1, and F4/80 and quantification of cell densities. Scale bar: 50 µm, Error bars, standard error of mean ** p<0.01, *** p<0.001.

### Detailed Description

The invention provides methods and compositions for use in or treating diseases and conditions of the eye by adoptive transfer of plasmacytoid dendritic cells (pDCs) to the eye. The methods and compositions of the invention can be used to prevent or treat diseases or conditions characterized by neovascularization of one or more tissues of the eye including, e.g., the cornea, the retina, or the choroid. The methods and compositions can also be used to prevent or treat diseases or conditions characterized by ocular (e.g., corneal) nerve degeneration or damage, as well as inflammation. Central to the invention are the discoveries that pDCs can be used to reduce or limit neovascularization, reduce or limit corneal nerve damage, promote corneal nerve regeneration, and prevent or reduce inflammation in the eye. The methods and compositions for use according to the invention are described further, as follows.

### Identification of Subjects

Subjects that can be treated using the methods and compositions for use according to the invention include those suffering from, or at risk for, neovascularization, nerve degeneration or damage, and/or inflammation of the eye. The subjects include human patients (adults and children) who have or are at risk of developing a disease or condition of the eye as described herein.

Neovascularization is a common feature of many conditions, and may occur in tissues of the eye including, for example, the cornea, retina, or choroid. This process involves new blood vessel formation in abnormal locations, such as the cornea, a normally avascular tissue. Diseases that are characterized by corneal neovascularization include, for example, corneal infection, inflammation, autoimmune disease, limbal stem cell deficiency, neoplasia, dry eye disease, radiation, blepharitis, uveitis, keratitis, corneal ulcers, glaucoma, rosacea, and lupus. Trauma, such as surgery, injury, burn (e.g., chemical burn), injury, and excessive or improper contact lens use, can also be characterized by neovascularization. Inflammation associated with ocular (e.g., corneal) neovascularization can result from bacterial and viral infection, Stevens-Johnson syndrome, graft rejection, ocular cicatricial pemphigoid, and degenerative disorders, such as pterygium and Terrien marginal degeneration. Diseases or conditions that are characterized by retinal neovascularization include, for example, ischemic retinopathies, diabetic retinopathy, retinopathy of prematurity, retinal vein occlusions, ocular ischemic syndrome, sickle cell disease, radiation, and Eales' disease. Further, diseases or conditions that are characterized by choroidal neovascularization include, for example, inflammatory neovascularization with uveitis, macular degeneration, ocular trauma, trauma due to excessive or improper contact lens wear, sickle cell disease, pseudoxanthoma elasticum, angioid streaks, optic disc drusen, extreme myopia, malignant myopic degeneration, and histoplasmosis. Subjects having or at risk of developing any of the aforementioned disorders or conditions can be treated using the methods and compositions of the invention.

The cornea is the most densely innervated structure in the human body, and is therefore highly sensitive to touch, temperature, and chemical stimulation, all of which are sensed by corneal nerves. Corneal nerves are also involved in blinking, wound healing, and tear production and secretion. Damage to or loss of corneal nerves can lead to dry eyes, impairment of sensation, corneal edema, impairment of corneal epithelium healing, corneal ulcerations and erosions, and a cloudy corneal epithelium, among other conditions. Diseases or conditions characterized by corneal nerve degeneration or damage include, for example, dry eye disease, neurotrophic keratitis, corneal infections, excessive or improper contact lens wear, ocular herpes (HSV), herpes zoster (shingles), chemical and physical burns, injury, trauma, surgery (including corneal transplantation, laser assisted in-situ keratomileusis (LASIK), penetrating keratoplasty (PK), automated lamellar keratoplasty (ALK), photorefractive keratectomy (PRK), radial keratotomy (RK), cataract surgery, and corneal incisions), abuse of topical anesthetics, topical drug toxicity, corneal dystrophies, vitamin A deficiency, diabetes, microbial keratitis, and herpetic keratitis (caused by, e.g., HSV-1). The methods and compositions for use according to the invention can be used to prevent or treat any of the aforementioned diseases or conditions of the eye.

Patients having or at risk of developing diseases or conditions characterized by inflammation within the eye can also be treated using the methods and compositions for use according to the invention. Thus, for example, patients having or at risk of the following diseases or conditions can be treated: episcleritis, scleritis, uveitis (e.g., anterior uveitis (including iritis and iridocyclitis), intermediate uveitis (including vitritis and pars planitis), posterior uveitis (including retinitis, choroiditis, chorioretinitis, and neuroretinitis), panuveitis (infectious) (including endophthalmitis), and panuveitis (non-infectious)), and retinal vasculitis.

### Plasmacytoid Dendritic Cells (pDCs)

The cells used in methods and compositions for use according to the invention are plasmacytoid dendritic cells (pDCs), which circulate in the blood and can also be found in peripheral lymphoid organs. pDCs are bone marrow-derived innate immune cells that express Toll-like receptors (TLR) 7 and 9. In mice, they express low levels of CD11c, which differentiates them from conventional dendritic cells (cDCs), and exhibit PDCA-1, Siglec-H, and CD45R/B220. In humans, pDCs are positive for blood-derived dendritic cell antigen (BDCA)-2 (CD303), BDCA-4 (CD304), and CD123. Upon activation, they produce large amounts of type 1 interferons (see, e.g., Tversky et al., Clin. Exp. Allergy 38(5):781-788, 2008; Asselin-Paturel et al., Nat. Immunol. 2(12):1144-1150, 2001; Nakano et al., J. Exp. Med. 194(8):1171-1178, 2001; Bjorck, Blood 98(13):3520-3526, 2001).

pDCs for use in the invention can be isolated from a subject to whom they are to be administered or they can be obtained from a donor (e.g., a human donor). pDCs can be isolated from blood or bone marrow using standard techniques including, e.g., density gradient centrifugation and marker-based cell separation. Optionally, the pDCs can be cultured and/or frozen prior to use. Furthermore, the pDCs can be obtained by the stimulation of cultured bone marrow cells. For example, peripheral blood mononuclear cells (PBMCs) can be isolated from blood using, e.g., Ficoll gradient density centrifugation. Then, pDCs can be isolated from PBMCs based on a pDC-specific or pDC-enriched marker (e.gBDCA-2, BDCA-4, or CD123). An antibody against such a marker (e.g., an anti-BDCA-2, anti-BDCA-4, or anti-CD123 antibody) can be used in this isolation step using standard methods (e.g., microbead or magnetic bead-based separation or fluorescence-activated cell sorting [FACS]).

In a specific example, 5-10 ml blood is collected from a subject via routine venipuncture and is placed in a tube containing citrate as an anti-coagulant. Next, PBMCs are separated by standard Ficoll density gradient centrifugation. After isolating PBMCs, pDCs are selected via commercially available magnetic beads according to the manufacturer's instructions (Miltenyi Biotec). In brief, PBMCs are blocked with an anti-Fc receptor antibody for 15 minutes at room temperature (RT). Next, samples are labeled by incubation with an anti-BDCA2 antibody conjugated with microbeads for 30 minutes at 4°C. Cells labeled with magnetic bead-conjugated BDCA-2 antibodies (which will constitute pDCs) are then applied to a separation column, placed in a separation device standing on a magnetic field. By washing the separation column with sterile washing buffer, BDCA2-negative cells (non-pDCs) are washed out, while BDCA-2⁺ labeled pDCs stay attached to the column. At this step, the separation column is removed from the magnetic field and pDCs are eluted by pushing washing buffer through the column. After separation, the number of pDCs is determined by routine Trypan blue staining on a portion of collected cells and the purity of the sample is measured by immunofluorescence staining with a BDCA2 fluorochrome-conjugated antibody (as well as other human pDC markers including BDCA-4 and CD123, if needed) and analyzed with FACS. In case analysis shows not satisfactory purity of the isolated cells (e.g., less than 85%), purity can be improved by another round of magnetic separation. Cells are then centrifuged and resuspended in sterile saline or tissue glue for adoptive transfer purposes.

### Compositions

The invention also includes compositions including pDCs as described herein, for use in the methods described herein. Such compositions include pDCs and a pharmaceutically acceptable carrier or diluent. For example, pDCs prepared, e.g., as described above, can be diluted or concentrated to a final concentration of, e.g., 10⁴-10⁸, 10⁵-10⁷, or 10⁶ cells per ml in a pharmaceutically acceptable carrier or diluent. The desired concentration of cells will vary depending on the method of administration and the type and severity of the disease or condition being treated. Depending upon the particular application, the carrier or diluent can be selected from, e.g., liquids, creams, drops, or ointments, as can be determined by those of skill in the art. For example, the cells can be administered by the use of a tissue adhesive or glue, such as a biologic adhesive (e.g., a fibrin-based adhesive or glue, such as Tisseel). Alternatively, a solution may be used (e.g., phosphate buffered saline, sterile saline, or sterile culture medium (e.g., RPMI or DMEM)). The cells may further be administered in the cell culture medium in which they were cultured. The compositions used in the invention typically include pDCs are at least 50% (e.g., at least 60%, 75%, 90%, 95%, 99%, or 100%) of the cells present in the compositions.

### Methods of Treatment

pDCs can be administered to the eye of a subject to be treated according to the methods of the invention using methods that are known in the art for ophthalmic administration. Different routes of administration are utilized, depending upon the part of the eye to be treated. For example, for treatment of a disease or condition of the cornea, direct topical application of a formulation (e.g., as described above) to the cornea can be used. In one example, isolated pDCs are diluted in tissue glue (e.g., Tisseel) at a density of 10⁶ cells/µl and applied to the cornea. If the corneal epithelium is not intact, the cells can be applied directly onto the cornea, but if the corneal epithelium is intact, it can be treated to make it permeable prior to administration of the cells. This can be achieved, for example, by the application of topical anesthetic eye drops or by mechanical abrasion or removal of corneal epithelium.

For treatment of a disease or condition of another part of the eye, e.g., the retina or the choroid, a different approach to administration may be selected. For example, intravitreal or sub-retinal injection may be utilized as determined to be appropriate by those of skill in the art. In a specific example, isolated pDCs are diluted in sterile culture media or phosphate buffered saline at a concentration of 10⁶ cells/µl, and administered to the retina or choroid by routine intravitreal or sub-retinal injection.

Treatment according to the methods of the invention can be carried out using regimens that are determined to be appropriate by those of skill in the art based on factors including, for example, the type of disease, the severity of disease, the results to be achieved, and the age and general health of the patient. Treatment according to the methods of the invention thus can take place just once, or can be repeated (e.g., 2, 3, 4, 5, 6, 7, 8, 9, 10, or more times). In the case of multiple treatments, appropriate intervals between treatments can be selected by those of skill in the art. The invention thus includes, e.g., hourly, daily, weekly, monthly, bi-monthly, semi-annual, or annual treatments.

Adoptive transfer of pDCs can be used to treating a disease or condition of the eye by preventing or reducing corneal, retinal, or choroidal neovascularization in a subject by, for example, 10% or more (e.g., at least 10%, 20%, 30%, 40%, 50%, 60%, 70%, 80%, 90%, or 100%) as compared to the amount of neovascularization observed before treatment. For example, neovascularization can be reduced by 25%, 50%, 2-fold, 5-fold, 10-fold or more, or is eliminated. Improvements in neovascularization may be assessed clinically by fundus examination and Optical Coherence Tomography (OCT) in patients, as is understood in the art.

In other examples, adoptive transfer of pDCs treats a disorder or condition of the eye by reducing nerve degeneration or damage (e.g., corneal nerve damage). Nerve regeneration (e.g., recovery from nerve damage) can be enhanced by, for example, 10% or more (e.g., at least 10%, 20%, 30%, 40%, 50%, 60%, 70%, 80%, 90%, or 100%) as compared to the baseline nerve density prior to treatment. For example, nerve regeneration can be enhanced by 25%, 50%, 2-fold, 5-fold, 10-fold or more. Corneal nerve damage may be assessed visually, i.e., by *in vivo* confocal imaging, or by restoration of function, such as increased tear production and secretion, improved wound healing, reduced pain, improved vision, and improved reflexes, such as the corneal blink reflex.

In further examples, adoptive transfer of pDCs treat a disorder or condition of the eye by reducing inflammation within or around the eye. Inflammation can be reduced by, for example, 10% or more (e.g., at least 10%, 20%, 30%, 40%, 50%, 60%, 70%, 80%, 90%, or 100%) as compared to the baseline inflammation prior to treatment.

In the case of prophylactic treatment, subjects at risk of developing a disease or condition of the eye, as described herein (e.g., subjects at risk for corneal, retinal, or choroidal neovascularization, ocular nerve degeneration or damage, and/or intraocular inflammation due to a disease or condition of the eye), may be treated prior to symptom onset or when symptoms first appear, to prevent development or worsening of neovascularization, degeneration, or damage. For example, in subjects already presenting with neovascularization, further growth of vessels into presently avascular tissue can be prevented by the methods of the present invention. Similarly, in subjects already presenting with nerve damage or degeneration, further damage or degeneration can be prevented by use of the methods and compositions for use according to the invention. Furthermore, in subjects already presenting with symptoms of intraocular inflammation, further inflammation can be prevented using the methods and compositions of the invention.

The following non-limiting examples are illustrative of the present disclosure.

### Examples

### Example 1: Neovascularization

### Presence of resident plasmacytoid dendritic cells in the naive murine cornea and their significant increase in density following induction of inflammation

To demonstrate the presence of corneal pDCs in steady state, we performed immunofluorescence (IF) staining on wild-type (WT) C57BL/6 (B6) mice corneal whole-mounts with fluorochrome-conjugated antibodies against Siglec-H (eBioscience, San Diego, CA), PDCA-1 (Miltenyi Biotec Inc., San Diego, CA; two specific murine pDC markers), and CD45 (pan-leukocyte marker; Biolegend, San Diego, CA). Briefly, corneas were excised (n=3-5), fixed for 15 minutes in chilled acetone, blocked for 60 minutes with 2% bovine serum albumin+1% FC block at room temperature (RT), incubated with antibodies overnight at 4°C and, after washing, mounted and imaged by a Leica TCS Spectral photometric SP5 laser confocal microscope.

To assess whether pDCs increased during inflammation, we used two well-established models of corneal suture placement and thermal cautery (Chen et al., Nat. Med. 10(8):813-815, 2004, Cursiefen et al., Proc. Natl. Acad. Sci. U.S.A. 103(30):11405-11410, 2006). Briefly, following topical application of ophthalmic proparacaine hydrochloride solution, three 11-0 nylon sutures (Surgical Specialties, Wyomissing, PA) were placed in the corneal periphery of anesthetized mice (100 mg/kg ketamine and 20 mg/kg xylazine). For thermal cautery, a fine diathermy tip (Fine Ophthalmic Tip, Aaron, St. Petersburg, FL) was placed on five separate points for 1 second each within the central 2 mm of the cornea of anesthetized mice. On day 3 following thermal cautery and on day 14 after suture placement, corneas were assessed with IF staining and confocal microscopy. Quantification was performed via Imaris (Bitplane AG, Zurich, Switzerland).

Fig. 1A (top panel) and Fig. 1B show the presence and density of pDCs (co-stained with CD45, Siglec-H, and PDCA-1) in naive corneas. Following induction of inflammation by suture placement (Fig. 1A, middle panel) or thermal cautery (Fig. 1A, bottom panel), there is a significant increase in corneal pDC density (Fig. 1B). These experiments demonstrate that the normal cornea is endowed with resident pDCs, and that inflammation results in a substantial increase in the density of corneal pDCs.

### Human corneas host resident plasmacytoid dendritic cells

In order to assess whether human corneas also harbor pDCs, we performed fluorescence activated cell sorting (FACS) on single cell suspensions of human corneas. Briefly, human corneas (Tissue Banks International, Baltimore, MD) were chopped and subjected to digestion in 2 mg/ml collagenase D and 0.5 mg/ml DNase (Sigma-Aldrich, St. Louis, MO) at room temperature for 30 minutes. Next, upon addition of FACS buffer to stop the reaction, digested corneas were filtered through a 40 µm cell strainer (Corning Inc., Corning, NY) to remove debris and undigested materials. Single cell suspensions were labeled with fluorochrome-conjugated antibodies against human BDCA2 and BDCA4 (two specific human pDC markers), CD45, or their respective isotypes (all Biolegend). Cells were then washed and analyzed with a BD LSR II Flow Cytometer. Further analysis was performed with Flowjo v9 (FlowJo LLC, Ashland, OR).

As shown in Fig. 2, pDCs were identified in human corneas as judged by co-expression of CD45, BDCA2, and BDCA4. Thus, human corneas host pDCs during steady state.

### Depletion of plasmacytoid dendritic cells is associated with breakdown of corneal angiogenic privilege and increased neovascularization during steady state and corneal inflammation

For local depletion of pDCs in corneas, we administered 30 ng diphtheria toxin (DT) subconjunctivally (s.c.) in transgenic BDCA2-DTR mice (called pDC-DTR from hereon). In these mice (established by Dr. Colonna, Washington University School of Medicine; obtained heterozygous through Jackson Laboratory and bred in house to homozygous) diphtheria toxin receptor (DTR) is inserted under the transcriptional control of a human C-type lectin domain family 4, member C (CLEC4C or BDCA2) promoter, allowing specific depletion of pDCs upon DT injection (Swiecki et al., Immunity. (2010) 33(6):955-66). For continuous depletion of pDCs, we repeated the s.c. DT injection every other day, as a single s.c. DT injection is effective for only 48 hours (Figs. 3A and 3B). Notably, long-term local depletion of pDCs is not associated with adverse health outcomes (Swiecki et al., Immunity 33(6):955-966, 2010; Mandl et al., PLoS One 10(8):e0134176, 2015; Rowland et al., J. Exp. Med. 211(10):1977-1991, 2014). Also, local DT is safe and does not affect nerve density and immune cell populations in the murine cornea (Hu et al., ARVO Meeting Abstracts 54:2158, 2013; Frank et al., J. Immunol. 188(3):1350-1359, 2012; Buela et al., J. Immunol. 194(1):379-387, 2015). After clinical scoring of neovascularization based on clock hours of neovascularized area, corneas were excised, IF staining was performed with CD31 (blood vessel endothelial marker; Biolegend), and whole-mounted corneas underwent confocal microscopy. Neovascular blood vessel length (Religa et al., Sci. Rep. 3:2053, 2013; Seo et al., Proc. Natl. Acad. Sci. U.S.A. 109(6):2015-2020, 2012) was measured on confocal micrographs using imaged (rsbweb.nih.gov/ij/). Age-matched WT B6 mice receiving s.c. DT served as controls. To evaluate whether pDC repopulation stimulates neovascular regression, after 7 days of pDC depletion, we allowed pDC repopulation for 14 days without DT injections and measured neovascularization. To assess whether pDCs limit angiogenesis during inflammation, pDCs were depleted and sutures were placed on the cornea the day after. pDC depletion was continued for 14 days after suture placement. Neovascularization was assessed clinically and by confocal microscopy.

Fig. 3 demonstrates successful depletion of pDCs upon local s.c. administration of DT in pDC-DTR mice. Figs. 4A-4M show that sham-depleted corneas do not stain with pan-endothelial marker CD31 due to corneal angiogenic privilege. However, depletion of pDCs is accompanied with rapid and severe breakdown of corneal angiogenic privilege. Furthermore, depletion of pDCs significantly augments neovascularization during inflammation as compared to controls. pDC repopulation results in regression of neovascularization. These findings show that pDCs play crucial roles in the maintenance of corneal vascular privilege, and that they limit the severity of corneal angiogenesis during inflammation.

### Depletion of plasmacytoid dendritic cells is accompanied by decreased mRNA levels of anti-angiogenic molecules endostatin and thrombospondin-1

On day 7 after suture placement in pDC-depleted NV or control corneas, total corneal RNA was extracted using an RNAeasy Mini kit (Qiagen, Valencia, CA). cDNA was synthetized using 300 ng RNA using a QuantiTect Reverse Transcription Kit (Qiagen) and relative mRNA levels of TSP-1 and endostatin, two anti-angiogenic molecules, were measured by qRT-PCR using iTaq^{™} Universal SYBR Green Supermix (Biorad Laboratories Inc., Hercules, CA).

Both TSP-1 and endostatin mRNA levels are significantly lower in the pDC-depleted NV corneas, as compared to WT B6 control mice after s.c. DT injections (p=0.01; Fig. 5). These results show that pDCs contribute to preservation of corneal angiogenic privilege by actively regulating secretion of anti-angiogenic molecules.

### Corneal plasmacytoid dendritic cells secrete endostatin

Seven-day sutured NV corneas were digested as described earlier for human corneas. A single cell suspension of corneas was then labeled with CD45 (pan-leukocyte marker), Siglec-H, PDCA-1, B220, (three molecules expressed by pDCs), endostatin (Abcam, Cambridge, MA), or isotype controls. Secondary antibody staining (for endostatin) was performed afterwards with anti-rabbit flourochrome-conjugated antibody (Jackson ImmunoResearch Laboratories, West Grove, PA). Cells were then washed and underwent FACS. These results show co-localization of endostatin with pDCs (Fig. 6), and show that pDCs have anti-angiogenic effects by actively secreting anti-angiogenic molecules.

### Vascularized tissues express higher levels of VEGF-A

Naive cornea, liver, and spleen were excised (n=3). Total RNA was extracted, cDNA was synthesized, and VEGF-A levels were measured by qRT-PCR. Higher levels of VEGF-A mRNA were observed in the liver and spleen, as compared to the cornea (Fig. 7). In vascularized tissues with resident pDCs, namely the spleen and liver, there are higher levels of VEGF-A, which may explain in part why these tissues are vascularized despite the presence of pDCs.

### Local adoptive transfer of plasmacytoid dendritic cells diminishes corneal neovascularization induced by suture placement

In order to assess the feasibility of local adoptive transfer of pDCs, we used transgenic DPE-GFP×RAG1^{-/-} mice with GFP⁺ pDCs (Iparraguirre et al., J. Leuk. Biol. 83(3):610-620, 2008) as pDC donors. To enhance pDC isolation yield, we injected 8-week old DPE-GFP×RAG1^{-/-} mice with 5 × 10⁶ B16 murine Flt3L-secreting melanoma tumor cells, as previously described (Bjorck, Blood. (2001) 98(13):3520-6; Brawand et al., J. Immunol. 169(12):6711-6719, 2002; Naik et al., Meth. Mol. Biol. 595:167-176, 2010). 10-14 days later, we harvested the spleens and sorted splenic GFP⁺ pDCs. By this method, we are able to sort 1-1.5×10⁶GFP⁺ pDCs from one animal. Next, following suture placement on corneas of WT B6 mice to induce NV, we debrided the central corneal epithelium mechanically (Johnson et al., Invest. Ophthal. Vis. Sci. 46(2):589-595, 2005) and applied 10⁴ GFP⁺ pDCs or PBS (control) on the cornea using TISSEEL tissue glue (Baxter Healthcare Corp.) (Zou et al., PLoS One 7(4):e34652, 2012; Thiebes et al., BioResearch Open Access 4(1):278-287, 2015). To assess the feasibility of local pDC adoptive transfer, we performed confocal microscopy on whole-mounted corneas 48 hours later. To evaluate the effect of local adoptive transfer of pDCs on corneal NV, we performed confocal microscopy as above, 7 days after suture placement and adoptive transfer.

As shown in Figs. 8A-8D, GFP⁺ pDCs were detected in the cornea 48 h following local adoptivetransfer. Further, adoptive transfer of pDCs led to reduced NV induced by suture placement (Figs. 8A-8D). These results show that local pDC adoptive transfer is feasible and efficacious in in reducing neovascularization in the murine cornea.

### The normal retina hosts resident plasmacytoid dendritic cells, which express endostatin

Naive retinas of 6-8 week old male WT B6 mice were excised and retinal single cells were obtained by digesting retinas using a method similar to that mentioned above for corneal FACS. A single cell suspension of retinal cells was then labeled with CD45, Siglec-H, PDCA-1, B220, and endostatin, washed, and analyzed with FACS. After gating out debris and doublets, CD45⁺PDCA-1⁺ cells were selected (Fig. 9A), showing CD45⁺PDCA-1⁺Siglec-H⁺B220⁺ pDCs (Fig. 9B). pDCs co-stained with endostatin (Fig. 9C). These data show that the retina have resident pDCs which express the anti-angiogenic molecule endostatin.

### Local depletion of retinal pDCs is accompanied by retinal NV and increased vascular permeability

Local pDC depletion in the retina was carried out by intravitreal injection of 30 ng (1-2 µl) DT with a 33-gauge needle (World Precision, Sarasota, FL) in pDC-DTR mice. The control group was WT B6 mice receiving DT. Injections were repeated every 48 hours to keep the retina devoid of pDCs. 0.1 mg/g 70 kD TRITC-dextran (Sigma-Aldrich) was injected intravenously (i.v.) to assess vascular permeability (Atkinson et al., Eye 6(Pt 4):440-446, 1991; Sun et al., J. Exp. Med. 209(7):1363-1377, 2012). In another set of experiments, following pDC depletion, retinas were stained with collagen IV (Abcam) followed by secondary antibody to assess NV, and underwent confocal microscopy. pDC depletion in the retina leads to NV and vascular leakage (Figs. 10A-10C). These results show that retinal pDCs, similar to corneal pDCs, show anti-angiogenic functions.

### Successful local adoptive transfer of plasmacytoid dendritic cells to naive retina

2×10⁴ GFP⁺ pDCs isolated (as described earlier) and transferred to naive WT B6 mouse retina without pDC depletion by intravitreal or subretinal injections (injection volume: 1-2 µl) (Westenskow et al., Journal of Visualized Experiments: JoVE. (2015) 95:52247; Siqueira et al., Retina. (2011) 31(6):1027-14; Park et al., Invest. Ophthal. Vis. Sci. (2015) 56(1):81-9; Wert et al., J. Vis. Exp.: JoVE 69, 2012). Control mice received intravitreal injection of PBS. 24 hours later, staining was performed with PDCA-1 and B220 on retinal single cell suspensions, followed by FACS. GFP⁺ pDCs were observed in the retina among non-GFP (host) pDCs after adoptive transfer (Fig. 11). These results show that adoptive transfer of pDCs to the naive retina is feasible by intravitreal or subretinal injections.

### Example 2: Nerve Regeneration

### Results

### Plasmacytoid Dendritic Cells Reside in Close Proximity to Sub-basal Nerve Plexus in Normal

### Cornea

As noted above, the cornea hosts resident pDCs under steady state. In order to study potential communication of pDCs with corneal nerves, we first assessed the spatial relation of pDCs and corneal nerves. As shown in Fig. 12, whole mount immunofluorescence (IF) staining of naive wild-type (WT) C57BL/6 cornea with βIII-tubulin (pan-neuronal marker; white), CD45 (pan-leukocyte marker; red), and PDCA-1 (pDC marker; green), reveals that pDCs lay in anterior stroma in close proximity to corneal sub-basal nerve plexus.

### Local Depletion of Corneal Plasmacytoid Dendritic Cells Is Accompanied by Abrupt Corneal Nerve Loss

Next, we depleted resident corneal pDCs by subconjunctival injection of 30 ng DT in transgenic BDCA2-DTR (pDC-DTR) mice. As previously mentioned, in these mice, diphtheria toxin receptor is expressed under transcriptional control of human BDCA2, a specific pDC gene. Therefore, in these transgenic mice pDCs are specifically ablated upon exposure to DT (Swiecki et al., Immunity 33(6):955-966, 2010). Also, we have shown that although single injection of DT is successful in depleting 80-90% of resident corneal pDCs, these cells are quickly repopulated in 3 days following injection. Thus, we repeated s.c. DT injections every 48 hours to keep cornea devoid of pDCs.

Upon pDC depletion, we assessed corneal blink reflex and subsequently nerve density on excised corneal whole-mounts by immunofluorescence staining followed by confocal microscopy. As shown in Figs. 12B-12D, we observed that pDC depletion is accompanied by severe degeneration of sub-basal and stromal nerve plexuses as early as day 1 following pDC depletion in both center (103.1±15.3 mm/mm² in sub-basal plexus and 17.1±2.4 in stromal plexus) and periphery (77.4±10.6 in sub-basal plexus and 24.6±8.0 in stromal plexus) of the cornea. Notably, degeneration of corneal nerves progressed during the course of experiments, as 7 days following pDC depletion, almost all corneal nerves in the center (1.1±0.7 mm/mm² in both plexuses combined) and periphery (5.3±3.9 in both plexuses combined) of the corneas were degenerated. In order to assess possible contribution of administration of DT or s.c. injection in pDC-DTR mice, we used two control groups in these experiments: WT C57BL/6 mice receiving s.c. 30 ng DT and pDC-DTR mice treated with similar volume of PBS. Notably, we did not observe any alterations in corneal nerve density following s.c. injection of PBS or DT in pDC-DTR and WT C57BL/6 mice, respectively. In agreement with this finding, we observed that the corneal blink reflex is diminished in the center of cornea upon pDC depletion (8.3% positive blink reflex on day 3, and 0% on day 7), however, this reflex remains intact in two control groups (frequency of positive blink reflex: 100%, p<0.001; Fig. 12E).

### Repopulation of Plasmacytoid Dendritic Cells Promotes Corneal Nerve Regeneration

Next, in order to study whether pDCs can induce nerve regeneration, we assessed corneal nerve regeneration after initial degeneration. For this experiment, we initially depleted pDCs in the cornea of pDC-DTR mice for 7 days to induce nerve degeneration. Next, we stopped DT injection to let pDCs repopulate in the cornea. 5 and 14 days following stopping DT injection, we measured corneal sub-basal and stromal nerve densities and observed substantial progressive regeneration of both plexuses in the center (31.8t on day 5 vs. 49.0± on day 14, p<0.001) and periphery (40.5± on day 5 vs. 81.8± on day 14, p<0.001) of cornea upon pDC repopulation (Fig. 13A). In line with this finding, we observed that 5 days after repopulation of corneas, corneal blink reflex is re-established in 19.3% of the mice and by day 14 following pDC repopulation, 93% of mice exhibit normal blink reflex (p<0.001; Fig. 13B).

### Plasmacytoid Dendritic Cells Are Vital Source of Nerve Growth Factor in Cornea

Furthermore, we studied the molecular mechanism orchestrating this observation. Considering numerous reports on the necessity of nerve growth factor (NGF) in maintenance and regeneration of peripheral nerves (Finn et al., J. Neurosci. 20(4):1333-1341, 2000; Patel et al., Neuron 25(2):345-357, 2000; White et al., J. Neurosci. 16(15):4662-4672, 1996), we assessed the mRNA levels of this neurotrophic molecule via qRT-PCR in corneal stroma, where pDCs reside, upon pDC depletion. As illustrated in Fig. 14A, we observed that NGF levels are decreased in the cornea following pDC depletion in pDC-DTR mice (0.18±0.02 fold change on day 7 following pDC depletion, p<0.001), however, its level reaches the levels of the steady state in naive WT C57BL/6 mice following pDC repopulation.

Next, in order to assess whether pDCs may present a source of NGF, we initially took advantage of transgenic DPE-GFP×RAG1^{-/-} mouse, with specifically GFP-tagged pDCs (lannacone et al., Nature 465(7301):1079-1083, 2010; IIparraguirre et al., J. Leukoc. Biol. 83(3):610-620, 2008). As shown in Fig. 14B, sorted splenic GFP-tagged pDCs of naive DPE-GFP×RAG1^{-/-} mice, were stained with NGF, suggesting that pDCs may serve as source of NGF. Next, in order to confirm that pDCs express NGF, we assessed presence of NGF mRNA in sorted GFP-tagged pDCs by reverse transcriptase PCR followed by PCR using NGF primer. Next, PCR products from the samples as well as controls lacking template RNA in cDNA synthesis step were subjected to agarose gels electrophoresis. As shown in Fig. 14C, sorted GFP-tagged pDCs from the spleen naive DPE-GFP×RAG1^{-/-} mice harbor endogenous NGF mRNA.

Further, we analyzed whether corneal pDCs can also produce NGF similar to splenic pDCs. As depicted in Fig. 14D, confocal micrograph of WT C57BL/6 mice showed that pDCs (CD45⁺PDCA-1⁺) co-stain with NGF (red) in the normal cornea. In order to validate this finding, we performed flow cytometry on single cell suspension of digested naive as well as inflamed corneas of WT C57BL/6 mice. For induction of inflammation, we applied corneal thermal cautery burn and suture placement, both of which are well-known techniques for sterile inflammation in cornea (Cursiefen et al., Proc. Natl. Acad. Sci. U.S.A. 103(30):11405-11410, 2006; Streilein et al., Invest. Ophthal. Vis. Sci. 37(2):413-424, 1996; Williamson et al., Invest. Ophthal. Vis. Sci. 28(9):1527-1532, 1987). We observed that corneal pDCs (identified by expression of CD45, PDCA-1, and B220) also co-stain with NGF in steady state as well as upon inflammation (Fig. 14E). Notably, in order to assure identifying pDCs accurately, we used two markers for pDCS (PDCA-1 and B220) in this experiment, as previous reports suggest that use of PDCA-1 as a single marker for identification of pDCs may encompass other cell entities including B cells, plasma cells, rare population of cDCs, as well as other immune cells, in particular following inflammation (Bao et al., Eur. J. Immunol. 41(3):657-668, 2011; Blasius et al., J. Immunol. 177(5):3260-3265, 2006; Vinay et al., J. Immunol. 184(2):807-815, 2010). In addition, increased levels of NGF mRNA were found in pDCs as compared to cDCs (Fig. 14F).

### Plasmacytoid Dendritic Cells Enhance Neurite Outgrowth in Trigeminal Ganglion Cell Culture through Secretion of Nerve Growth Factor in vitro

We further assessed whether pDCs secrete functionally active NGF. We cultured isolated trigeminal ganglion cells (TGCs) for one day and then added different numbers of sorted splenic GFP-tagged pDCs from naive DPE-GFP×RAG1^{-/-} mice to transwells to conduct a co-culture study. We assessed neurite outgrowth on TGCs, 3 days after co-culture and observed a considerable increase in the length of TGC neurites in parallel to density of pDCs in transwells (Fig. 15A and 15B).

To further confirm our finding, we also measured expression of neuro-regenerative markers including small proline-rich repeat protein 1a (Sprr1a), growth-associated protein-43 (Gap-43), vimentin, and brain derived neurotrophic factor (BDNF) (Pernet et al., Cell Death Differ. (2012) 19(7):1096-108; Bonilla et al., J. Neurosci. 22(4):1305-1315, 2002; Sun et al., Nature 480(7377):372-375, 2011; Sarkar et al., Invest. Ophthal. Vis. Sci. 54(9):5920-5236, 2013) in cultured TGCs. As demonstrated in Fig. 15C, in line with our neurite outgrowth measurement, we observed higher expression of neuro-regenerative markers in cultured neurons along with increase in the density of pDCs in the system.

Next, to study if pDCs secrete NGF *in vitro,* we measured the level of NGF in the co-culture media. We noted substantial increase in the amount of NGF in the media in conditions of culturing TGCs with pDCs versus TGCs alone. Interestingly, the increase in NGF was dependent on pDCs as similar amounts of NGF were detected in the cell culture media of pDC and TGC co-culture versus pDC monoculture in transwells (Fig. 15D).

### Experimental Methods

### Animals

Six- to ten-week-old male wild-type (WT) C57BL/6 mice were purchased from Charles River (Charles River Laboratories International, Wilmington, MA); DPE-GFP×RAG1^{-/-} mice, with specifically GFP-tagged pDCs (lannacone et al., Nature 465(7301):1079-1083, 2010; Iparraguirre et al., J. Leukoc. Biol. 83(3):610-620, 2008), and BDCA2-DTR mice (C57BL/6 background; Jackson Laboratory, Bar Harbor, ME) (Swiecki et al., Immunity 33(6):955-966, 2008) were bred in house in specific pathogen free conditions. BDCA2-DTR mouse were bred to homozygousity for the experiments. For culturing TGCs, 10 day old transgene negative pups were used. All protocols were approved by Schepens Eye Research Institute, and Tufts Medical Center and Tufts University School of Medicine Animal Care and Use Committees (IACUC), and animals were treated according to the Association for Research in Vision and Ophthalmology (ARVO) Statement for the Use of Animals in Ophthalmic and Vision Research.

### Assessment of Corneal Sensation

Corneal blink reflex was assessed as previously described (Yamaguchi et al., PLoS One 8(8):e70908, 2013). In brief, an 8-0 nylon thread was applied to the central cornea of un-anesthetized mice under direct vision through a dissecting microscope to avoid contact with whiskers and eyelashes. The procedure was repeated three times on each mouse to ensure reproducibility.

### Corneal Immunofluorescence Staining, Confocal Microscopy, and Image Quantification

For immunofluorescent staining with NGF, corneal epithelium was removed with fine forceps following incubating corneas with 20 mM EDTA (Sigma-Aldrich) at 37°C for 30 minutes, as previously described (Hamrah et al., Invest. Ophthal. Vis. Sci. 43(3):639-646, 2002). Excised whole corneas or corneal stromas were fixed with chilled acetone (Sigma-Aldrich) at -20°C for 15 minutes. After washing fixed samples with PBS 3 times, samples were blocked in 2% bovine serum albumin (BSA; Sigma-Aldrich) and 1% anti-CD16/CD32 Fc receptor (FcR) mAb (2.4G2; Bio X Cell, West Lebanon, NH) for 30 minutes at room temperature. Next, samples were stained with fluorophore-conjugated CD45 (BioLegend), PDCA-1 (Miltenyi Biotec Inc., San Diego, CA), PIII-Tubulin (R&D Systems, Minneapolis, MN), or biotinylated anti-NGF (BioLegend) antibodies overnight at 4°C. Following three washes with PBS, if needed, samples were incubated with secondary anti-biotin antibody (BioLegend), for 1 hour at room temperature. Next, after washing with PBS 3 times, corneas were mounted with Vectashield with DAPI (Vector Labs, Burlingame, CA) and underwent microscopy via upright TCS SP5 Leica confocal microscope (Leica Microsystems, Germany). For quantification purposes, 3 images from the periphery and a single image from the center of the cornea were taken. Quantification of nerve density was performed via NeuronJ plugin (Meijering et al., Cytometry A. 58(2):167-176, 2004) for imaged software (NIH, Bethesda, MD), as previously described (Yamaguchi et al., PLoS One 8(8):e70908, 2013; Hu et al., PLoS One 10(9):e0137123, 2015).

### Corneal Single Cell Suspension and Flow Cytometry

Corneas were digested to yield single cells as previously described (Hamrah et al., Invest. Ophthal. Vis. Sci. 44(2):581-589, 2003). In brief, naive and inflamed corneas were excised (n=12 for naïve and n=5 for inflamed groups), cut into small pieces, and digested with 2 mg/ml collagenase D (Roche, Indianapolis, IN) and 0.05 mg/ml DNAse (Roche, Indianapolis, IN) for 45 minutes at 37°C in a humidified atmosphere with 5% CO₂. Next, digested corneas passed through a 40 mm cell strainer (BD Falcon, Becton-Dickinson, Franklin Lakes, NJ) to remove undigested materials. Next, single corneal cells were washed, blocked with 1% anti-CD16/CD32 FcR mAb (Bio X Cell) in 0.5% BSA containing 0.5% Tween 20 (Sigma-Aldrich) for 20 minutes at room temperature, and stained with combinations of antibodies against CD45, CD11c, CD11b, F4/80, PDCA-1, CD45R/B220, NGF, or their respective isotype controls (all BioLegend except for CD11c, from BD Bioscience, San Jose, CA) for 30 minutes in FACS buffer at room temperature in the dark. After washing with PBS, samples were incubated with secondary antibody against biotin (Jackson ImmunoResearch Laboratories, Inc., West Grove, PA) for 30 minutes at room temperature. Afterwards, samples were washed and reconstituted in 4% paraformaldehyde and underwent data acquisition with a BD LSR II flow cytometer (BD Biosciences). Data were analyzed with FlowJo V9.2 (FlowJo, LLC). Forward and side scatter plots were used to exclude dead cells, debris, and doublets.

### Splenic pDC Isolation

pDCs were isolated from DPE-GFP×RAG1^{-/-} mice. DPE-GFP×RAG1^{-/-} mice underwent subcutaneous injection of 5 × 10⁶ B16 murine Flt3L-secreting melanoma tumor cells. 10-14 days later, mice were euthanized. Spleens were harvested, and mechanically disturbed using a 5 ml syringe plunger and were filtered through a 40 mm cell strainer (BD Falcon). Next, after incubation with ice-cold ammonium chloride (ACK) lysis buffer (Biofluids, Rockville, MD) for 1 minute to remove contaminating RBCs, cells were washed with PBS. GFP-tagged pDCs were sorted via Moflo Cell Sorter (Beckman Coulter, Brea, CA).

### pDC and TGC Co-culture and Microscopy

Initially, 10 day old pups were euthanized, TGs were excised, chopped into small fragments, and digested in 2 mg/ml Collagenase D (Roche), 2 mg/ml DNAse I (Roche), and 5 mg/ml Dispase II (Sigma-Aldrich) in Hank's Balanced Salt Solution (Gibco) at 37°C for 30 minutes. Next, after filtering, cells were layered over a 12.5% on 28% Percoll (GE Healthcare, Pittsburgh, PA) gradient in L15 media (Gibco) and centrifuged at 1300 g for 10 minutes. Following removing debris in the percoll interface, purified TGCs were recovered from the bottom of the gradient. Next, 10,000 cells/well were seeded in 24 well cell culture plates coated with growth factor reduced Matrigel (Corning Inc, Corning, NY) in Ham's F-12 Nutrient Mix (Gibco) supplemented with 10% heat inactivated FBS (Gemini Bioproducts), 1% penicillin/streptomycin (Life Technologies), and 100 ng/ml NGF (Sigma-Aldrich). After one day of culture, media was changed to a similar media without NGF and sorted pDCs with different numbers were added to transwells. On day 3 following co-culture, transwells were removed, TGCs were stained with 1 µM Calcein (Life Technologies) and underwent imaging by an inverted Nikon Eclipse Ti inverted microscope (Nikon Inc., Melville, NY) equipped with an Andor Clara E digital camera (Andor Technology Ltd., Belfast, UK). Three images were taken from each well. Further, cell culture media was collected and kept in - 80°C for further protein measurement. TGCs were used for RNA extraction and quantitative real-time PCR.

### RNA Isolation, cDNA Synthesis, and Semi-Quantitative Real-time PCR

Corneal epithelium was removed with fine forceps following 30 minutes incubation with PBS containing 20 mM EDTA (Sigma-Aldrich) at 37°C. Next, 4-6 corneal stromas were pooled and lysed using BeadBug Microtube Homogenizer (Benchmark Scientific, Inc., Edison, NJ). Next, RNA was isolated from the corneal stroma using RNeasy Plus Universal Mini kit (QIAGEN, Germantown, MD). For isolating RNA from freshly sorted pDCs, purified cDCs, cultured pDCs, and cultured TGCs, RNeasy Plus Micro Kit (QIAGEN) was used. RNA yield was measured by spectroscopy (NanoDrop ND-1000; NanoDrop Technologies, Inc., Wilmington, DE). cDNA was synthetized using 300 ng of template RNA using QuantiTect Reverse Transcription kit (Qiagen). qRT-PCR was performed using iTaq Universal SYBR Green Supermix (Biorad, Hercules, CA) and Eppendorf Mastercycler RealPlex 2 (Eppendorf, Hauppauge, NY) with the primers set forth in Table 1. Relative mRNA level was measured with ΔΔCT method.

**Table 1. Primers**

| Transcript | Forward | Reverse |
|---|---|---|
| Endostatin | 5'-GCCCAGCTTCATCACAGAGT-3' (SEQ ID NO: 1) | 5'-TGTTGAAAGATGACTGGCTG-3' (SEQ ID NO: 2) |
| Thrombospondin-1 | 5'-TGGCCAGCGTTGCCA-3' (SEQ ID NO: 3) | 5'-TCTGCAGCACCCCCTGAA-3' (SEQ ID NO: 4) |
| NGF | 5'-AGCATTCCCTTGACACAG-3' (SEQ ID NO: 5) | 5'-GGTCTACAGTGATGTTGC-3' (SEQ ID NO: 6) |
| Sprr1a | 5'-GAACCTGCTCTTCTCTGAGT-3' (SEQ ID NO: 7) | 5'-AGCTGAGGAGGTACAGTG-3' (SEQ ID NO: 8) |
| Gap-43 | 5'-TGCTGTCACTGATGCTGCT-3' (SEQ ID NO: 9) | 5'-GGCTTCGTCTACAGCGTCTT-3' (SEQ ID NO: 10) |
| Vimentin | 5'-TACAGGAAGCTGCTGGAAGG-3' (SEQ ID NO: 11) | 5'-TGGGTGTCAACCAGAGGAA-3' (SEQ ID NO: 12) |
| BDNF | 5'-CAAAGCCACAATGTTCCACCAG-3' (SEQ ID NO: 13) | 5'-GATGTCGTCGTCAGACCTCTCG-3' (SEQ ID NO: 14) |
| GAPDH | 5'- CCCACTAACATCAAATGGGG-3' (SEQ ID NO: 15) | 5'- GATGATGACCCTTTTGGCTC-3' (SEQ ID NO: 16) |

### pDC and TGCs Co-culture Media ELISA

NGF levels in culture media of pDC monoculture or TGC and pDC co-culture were measured by ChemiKine Nerve Growth Factor Sandwich ELISA (Millipore, Billerica, MA).

### Statistical Analysis

Data was analyzed with SPSS version 17 (SPSS Inc., Chicago, IL). T test and ANOVA with Bonferroni or LSD host hoc were applied to assess differences among two or more groups, respectively, if assumptions were met. Chi square was used to compare categorical data. p less than 0.05 was considered significant.

### Subconjunctival Injections

Mice were anesthetized with intraperitoneal (i.p.) injection of 100 mg/kg ketamine and 10-20 mg/kg Xylazine. After application of topical proparacaine hydrochloride, 30 ng DT (Sigma-Aldrich St. Louis, MO) in 10 µl PBS was administered subconjuctivally by means of a Nanofil syringe with 33-gauage needle to BDCA2-DTR mice to locally deplete pDCs. Injections were repeated every 48 hours to keep corneas pDC-depleted. WT C57BL/6 mice receiving DT and BDCA2-DTR mice receiving PBS served as control groups. Erythromycin ophthalmic ointment was applied on eye after injections. Mice were randomly assigned to study groups using a Random Number Table.

### Corneal Suture Placement

Under deep anesthesia and following application of topical proparacaine hydrochloride, corneal suture placement was performed on WT C57BL/6 mice as previously described (Cursiefen et al., Proc. Natl. Acad. Sci. U.S.A. 103(30):11405-11410, 2006; Streilein et al., Invest. Ophthal. Vis. Sci. 37(2):413-424, 1996). Briefly, three 11-0 nylon sutures (Sharpoint; Vanguard, Houston, TX) were placed through the paracentral stroma of WT C57BL/6 mice, each 120° apart, without perforating the cornea, using aseptic microsurgical technique and an operating microscope.

### Corneal Thermal Cautery

As described previously (Streilein et al., Invest. Ophthal. Vis. Sci. 37(2):413-424, 1996; Williamson et al., Invest. Opthal. Vis. Sci. 28(9): 1527-1532, 1987), five light burns were induced on the central 50% of the cornea of deeply anesthetized WT C57BL/6 mice after topical treatment with proparacaine hydrochloride, via the tip of a handheld thermal cautery (Aaron Medical Industries Inc., St. Petersburg, FL) under a dissecting microscope.

### Agarose Gel Electrophoresis

RNA extraction and cDNA synthesis was performed as described on GFP-tagged pDCs from the spleen naive DPE-GFP×RAG1^{-/-} mice. PCR was performed under similar conditions described under qRT-PCR section using NGF primers. PCR products were run on 2% agarose gel. Gels were cast using 2% agarose (Sigma-Aldrich) in 0.5x Tris/borate/EDTA (TBE buffer) supplemented with 10 mM MgCl₂ and 0.5 mg/ml ethidium bromide (Sigma-Aldrich).

### Example 3: pDCs Induce Nerve Regeneration after Corneal Nerve Damage

### Results

### Local Adoptive Transfer of Plasmacytoid Dendritic Cells as a Therapeutic Approach for Corneal Nerve Regeneration

Corneas of 6-8-week-old male wildtype (WT) C57BL/6 mice underwent deep stromal trephination with a 2 mm trephine to sever corneal nerves. Splenic GFP⁺ pDCs from DPE-GFP×RAG1^{-/-} mice and WT CD11b⁺ myeloid cells were isolated. After trephination, 10⁴ pDCs, CD11b⁺ cells, or PBS control were locally applied onto the corneas using Tisseel tissue glue. On day 3, corneas underwent flow cytometry to assess protein expression of NGF. On day 14, corneas were stained for βIII-tubulin (pan-neuronal marker), CD45 (pan-leukocyte marker), and MHC-II (maturation marker). Total length of corneal nerves was quantified via NeuronJ and densities of MHC-II⁺ cells were measured by imaged. ANOVA with LSD post hoc test was used to assess statistical significance. p<0.05 was considered significant.

Confocal microscopy confirmed successful transfer of GFP⁺ pDCs to both central (331.5±42.7 cells/mm²) and peripheral (447.9±74.5) corneas on day 1 following local application of pDCs. Flow cytometry showed a 1.4-fold increase in the density of NGF⁺ cells on day 3 following adoptive transfer of pDCs, as compared with Tisseel-only control. One-time adoptive transfer of pDCs was accompanied by enhanced nerve regeneration on day 14 post-trephination in both the center (44.5±10.1 mm/mm²) and periphery (75.9t10.9) of corneas, compared with transfer of CD11b⁺ cells (24.9±11.7, p=0.02 in center and 47.7±8.2, p=0.002 in periphery) as well as Tisseel-only controls (22.2±6.3, p=0.005 in center and 62.3±4.0, p=0.04 in periphery). In corneas treated with local pDC transfer, we observed no significant increase in the density of MHC-II expressing leukocytes in the center (188.3:t32.1 cells/mm² vs. 246.4±61.4 in Tisseel-only and 301.7t68.2 in CD11b⁺ cell-treated) or periphery (205.4±24.4 vs. 250.8±18.3 in Tisseel-only and 239.8±23.8 in CD11b⁺ cell-treated) as compared with control groups (p>0.05), suggesting safety of local pDC adoptive transfer.

These results show that local adoptive transfer of pDCs can enhance corneal nerve regeneration following nerve damage and can serve as a cell-based therapeutic approach to treat corneal nerve damage.

### Experimental Methods

### Animals

Six- to ten-week-old male wild-type (WT) C57BL/6 mice were purchased from Charles River (Charles River Laboratories International); DPE-GFP×RAG1^{-/-} mice, with specifically GFP-tagged pDCs (lannacone et al., Nature 465(7301):1079-1083, 2010; Iparraguirre et al., J. Leukoc. Biol. 83(3):610-620, 2008) were bred in house in specific pathogen free conditions. All protocols were approved by Schepens Eye Research Institute, and Tufts Medical Center and Tufts University School of Medicine Animal Care and Use Committees (IACUC), and animals were treated according to the Association for Research in Vision and Ophthalmology (ARVO) Statement for the Use of Animals in Ophthalmic and Vision Research.

### Splenic Plasmacytoid Dendritic Cell and CD11b⁺ Myeloid Cell Isolation

Splenic GFP⁺ pDCs were sorted from DPE-GFP×RAG-1^{-/-} mice and CD11b⁺ myeloid cells were isolated from WT C57BL/6 mice. To enhance pDC isolation yield, we injected 8-week old DPE-GFP×RAG1^{-/-} mice with 5 × 10⁶ B16 murine Flt3L-secreting melanoma tumor cells, as previously described (Bjorck, Blood 98(13):3520-3526, 2001; Brawand et al., J. Immunol. 169(12):6711-6719, 2002; Naik et al., Meth. Mol. Biol. 595:167-176, 2010). 10-14 days later, we harvested the spleens and sorted GFP⁺ pDCs. Briefly, spleens of tumor-bearing DPE-GFP×RAG1^{-/-} or naive WT C57BL/6 mice were harvested, mechanically dissociated, and passed through a 40 µm cell strainer (BD Falcon) to yield single cell suspensions of splenic cells. Next, RBCs were lysed using ACK RBC lysis buffer (Biofluids). GFP⁺ pDCs were sorted using MoFlo Astrios EQ (Beckman Coulter) and CD11b⁺ cells were isolated using CD11b MicroBeads isolation kit (Miltenyi Biotec).

### Corneal Trephination and Local Adoptive Transfer of plasmacytoid Dendritic Cells

WT C57BL/6 mice were anesthetized with i.p. injection of 100 mg/kg Ketamine and 10-20 mg/kg Xylazine. After application of topical proparacaine hydrochloride, corneas were trephined using a 2 mm trephine and central corneal epithelium was debrided using an Algerbrush II corneal rust ring remover with a 0.5-mm burr (Alger Equipment Co, Lago Vista, TX). 10⁴ isolated splenic pDCs or CD11b⁺ cells were placed on the center of corneas using TISSEEL fibrin sealant (Baxter Healthcare Corporation, Deerfield, IL). Mice receiving tissue fibrin sealant only served as controls.

### Immunofluorescence Staining and Confocal Microscopy

GFP⁺ pDC-transferred corneas were excised, mounted with DAPI-containing medium (Vector Laboratories Inc.), and imaged by a Leica TCS SP8 (Leica Microsystems, Wetzlar, Germany) confocal microscope to assess presence of adoptively-transferred pDCs in the cornea. 14 days following trephination and adoptive transfer, corneas were harvested, fixed in chilled acetone (Sigma-Aldrich), blocked in 2% bovine serum albumin (BSA; Sigma-Aldrich) and 1% anti-CD16/CD32 Fc receptor (FcR) mAb (2.4G2; Bio X Cell) for 30 minutes at RT, and incubated with combinations of fluorochrome-conjugated primary Abs including MHC-II (both BioLegend) and βIII-tubulin (R&D Systems) overnight at 4°C. After washings, samples underwent confocal microscopy. For quantification purposes, 2-3 images from the periphery and a single image from the center of the cornea were taken. Quantification of nerve density was performed via NeuronJ plugin (Meijering et al., Cytometry A 58(2):167-176, 2004) for imaged software (NIH, Bethesda, MD), as previously described (Yamaguchi et al., PLoS One 8(8):e70908, 2013; Hu et al., PLoS One 10(9):e0137123, 2015). Cell densities were quantified via IMARIS (Bitplane AG).

### Corneal Single Cell Suspension and Flow Cytometry

Corneas were excised, cut into pieces and digested via incubation with 2 mg/ml collagenase D (Roche, Indianapolis, IN) and 0.05 mg/ml DNAse (Roche) to yield single cells prior to flow cytometric analysis. Next, after blocking, samples were labeled with biotin-labeled NGF antibody or its respective isotype control (both BioLegend). Samples were then washed and after staining with anti-biotin secondary Ab (Jackson ImmunoResearch Laboratories), washed, and analyzed with a BD LSR II flow cytometer (BD Biosciences, San Jose, CA). Data were analysed with FlowJo V9.2 (FlowJo, LLC, Ashland, OR). Forward and side scatter plots were used to exclude dead cells, debris, and doublets.

### Statistical Analysis

Data was analyzed with SPSS version 17 (SPSS Inc., Chicago, IL). ANOVA with LSD host hoc was applied to assess differences among groups. p less than 0.05 was considered significant.

### Example 4: Adoptive Transfer of pDCs to Ameliorate Corneal HSV-1 Keratitis

### Results

### Local Depletion of plasmacytoid Dendritic Cells Enhances Severity of Herpes Simplex Virus-1 Keratitis

We depleted resident corneal pDCs by subconjunctival injections of 30 ng DT in pDC-DTR mice as above. Control groups consisted of WT C57BL/6 mice receiving subconjuctival DT and pDC-DTR mice treated with subconjunctival PBS (referred to as sham-depleted in this section). Subsequently, we induced Herpes Simplex Virus-1 (HSV-1) keratitis by inoculating 2×10⁶ PFU HSV-1 McKrae strain after scarifying the corneas. Local pDC depletion enhanced the severity of HSV-1 keratitis judged by clinical assessment of corneal opacity as early as day 1 following HSV-1 inoculation (Fig. 17A). Consistent with clinical assessments, confocal microscopy of whole-mounted corneas stained with CD45 (pan-leukocyte marker), Gr-1 (neutrophil marker), and F4/80 (macrophage marker) showed significant increase of recruited leukocytes (5-fold at day 3), neutrophils (3.5-fold at day 3), and macrophages (5-fold at day 3) compared with sham-depleted controls (Fig. 17B).

### Local Depletion of plasmacytoid Dendritic Cells Is Accompanied by Decreased Levels of Interferon-α and Transforming Growth Factor-β1 in the Cornea during Herpes Simplex Virus-1 Keratitis

Next, we assessed mRNA and protein levels of Interferon-a (IFN-α) and Transforming Growth Factor-β1 (TGF-β1) in the corneal stroma of the pDC- and sham-depleted mice on day 3 in acute HSV-1 keratitis. We observed that local pDC depletion leads to decreased levels of IFN-α and TGF-β1 in the whole corneas during acute HSV-1 keratitis (Fig. 18A), suggesting the role of pDCs in their production in the cornea. In order to determine if corneal pDCs are the source of IFN-α as well as TGF-β1, we performed single cell qRT-PCR on corneal GFP⁺ pDCs of DPE-GFP×RAG1^{-/-} mice 24 hours after topical application of 10 µg Imiquimod (TLR7 agonist [Lee et al., Proc. Natl. Acad. Sci. U.S.A. 100(11):6646-51, 2003; Miller et al., Intl. J. Immunopharm. 21(1):1-14, 1999)]), 10 µg CpG 1826 oligonucleotide (CpG-ODN; TLR9 agonist), or control ODN. We observed that corneal pDCs produce IFN-α and TGF-β1 mRNAs in normal state and after stimulation with either TLR7 or TLR9 agonists, they increase their mRNA levels of IFN-α and TGF-β1 (>10 fold and >1000 fold, respectively; Fig. 18B). To confirm production of TGF-β1 by corneal pDCs, we subjected naive corneas to flow cytometric evaluation. We observed that pDCs while only 6% of non-pDC CD45⁺ resident leukocytes co-express TGF-β1, 58% of corneal pDCs co-stain with this molecule (Fig. 18C).

### Interferon-α and Transforming Growth Factor-β1 Are Vital in Modulating Immune Response in Herpes Simplex Virus-1 Keratitis

In order to evaluate the importance of IFN-α during HSV-1 keratitis, we performed local IFN-α blockade by application of anti-IFN-α Ab and studied the severity of HSV-1 keratitis clinically and measured the density of immune cell infiltration. We observed that local IFN-α blockade enhances corneal opacity in HSV-1 infected corneas compared with controls receiving normal saline (1.5 vs. 0.8 on day 3, p<0.05; Fig. 19A). Further, we observed an increase in the density of immune cells in the corneas including CD45⁺ leukocytes (2.8-fold), Gr-1⁺ neutrophils (10-fold), and F4/80⁺ macrophages (3.7-fold; Fig. 19B) on day 3 of HSV-1 keratitis in corneas with IFN-α blockade. These findings suggest that decreased IFN-α following pDC depletion may in part explain the observed increase in the density of recruited leukocytes and severity of clinical disease in HSV-1 keratitis.

Similarly, to assess the role of TGF-β1 in HSV-1 keratitis, we locally blocked corneal TGF-β1 by means of TGF-β neutralizing Ab. We observed that TGF-β1 blockade leads to increased severity of the corneal opacity (1 vs. 2 on day 3, p<0.05; Fig. 20A) and accompanies enhanced recruitment of immune cells including CD45⁺ cells (2 fold), F4/80 macrophages (6 fold), and Gr-1⁺ neutrophils (5 fold) in HSV-1 infected corneas at day 3 compared to controls treated with normal saline (Fig. 20B). These results suggest that pDCs possess an immune-modulatory effect through secretion of IFN-α and TGF-β1.

### Local Adoptive Transfer of plasmacytoid Dendritic Cells Ameliorates Herpes Simplex Virus-1 Keratitis

Observing critical role of pDCs in minimizing severity of corneal manifestations in acute HSV-1 keratitis, we evaluated if local adoptive transfer of pDCs can diminish severity if corneal signs and enhance viral clearance. Thus, we debrided epithelium of central cornea and adoptively transferred 10⁴ sorted splenic pDCs using fibrin sealant as described above. 24 hours following adoptive transfer, we inoculated 2×10⁶ PFU HSV-1 on the corneas (n=4-6/group). We observed that adoptive transfer of pDCs is accompanied by less clinically severe disease (0.2 ± 0.5; Fig. 21A-B) compared with sham-transferred controls (2.0 ± 0.6; p=0.002) on day 5 following inoculation of HSV-1. We next assessed IFN-α level in the cornea. As presented in Fig. 21C, we observed that adoptive transfer of pDCs to cornea is associated with higher levels of IFN-α mRNA in the corneal stroma (2.9 ± 0.8-fold change; p=0.001). Consistent with the clinical findings, we observed that adoptive transfer of pDCs enhances viral clearance evidenced by lower HSV-1 glycoprotein B (gB) RNA load (0.2 ± 0.2-fold change; p=0.009) in the cornea following adoptive transfer (Fig. 21D).

### Experimental Methods

### Animals

Six- to ten-week-old male wild-type (WT) C57BL/6 mice were purchased from Charles River (Charles River Laboratories International, Wilmington, MA); DPE-GFP×RAG1^{-/-} mice, with specifically GFP-tagged pDCs (lannacone et al., Nature 465(7301):1079-1083, 2010; Iparraguirre et al., J. Leukoc. Biol. 83(3):610-620, 2008), and BDCA2-DTR mice (C57BL/6 background; called pDC-DTR; Jackson Laboratory, Bar Harbor, ME) (Swiecki et al., Immunity 33(6):955-966, 2010) were bred in house in specific pathogen free conditions. pDC-DTR mouse were bred to homozygousity for the experiments. All protocols were approved by Schepens Eye Research Institute, and Tufts Medical Center and Tufts University School of Medicine Animal Care and Use Committees (IACUC), and animals were treated according to the Association for Research in Vision and Ophthalmology (ARVO) Statement for the Use of Animals in Ophthalmic and Vision Research.

### Acute Herpes Simplex Virus Keratitis Model

HSV-1 strain McKrae (kindly provided by Dr. Homayon Ghiasi, Cedars-Sinai Medical Center, Los Angeles, CA), a neurovirulent, stromal disease-causing strain, was used for ocular challenge (Sawtell et al., J. Virol. 72(7):5343-5350, 1998; Ghiasi et al., Virus Res. 65(2):97-101, 1999; Jiang et al., MBio 6(6):e01426-15, 2015). Mice were anesthetized with i.p. injection of 100 mg/kg Ketamine and 10-20 mg/kg Xylazine. After application of topical proparacaine hydrochloride, corneas were scarified using a 30-gauge needle; next, corneas were inoculated topically with 2×10⁶ PFU of HSV-1 strain McKrae in DMEM culture media (Mediatech, Inc, Manassas, VA).

### Clinical Evaluation of Herpes Simplex Keratitis Severity

The severity of acute keratitis was assessed by a blinded observer by slit-lamp bio-microscopy of corneas as previously described (Hu et al., PLoS One 10(9):e0137123, 2015; Inoue et al., Invest. Ophthal. Vis. Sci. 41(13):4209-4215, 2000). Briefly, corneal opacification were scored using the following scoring: 0, normal; 1, corneal opacity confined to less than one quarter of the cornea with visible iris; 2, corneal opacity between one quarter and one half of the cornea with visible iris; 3, corneal opacity extended to greater than half of the cornea with partially invisible iris; and 4, maximal corneal opacity spread over the entire cornea and completely invisible iris.

### Subconjunctival Injections

Mice were anesthetized with i.p. injection of 100 mg/kg Ketamine and 10-20 mg/kg Xylazine. After application of topical proparacaine hydrochloride, 30 ng DT (Sigma-Aldrich) in 10 µl PBS was administered subconjuctivally by means of a Nanofil syringe with 33-gauage needle to pDC-DTR mice to locally deplete pDCs. Injections were repeated every 48 hours to keep corneas pDC-depleted. WT C57BL/6 mice receiving DT and pDC-DTR mice receiving PBS served as control groups. For IFN-α or TGF-β1 blockade, 10 µg of INF-α (Hycult Biotech Inc., Plymouth Meeting, PA), TGF-β1 (Thermo Fisher Scientific, Waltham, MA) neutralizing antibodies, or normal saline was administered subconjuctivally to WT C57BL/6 mice and injections were repeated every 48 hours. Erythromycin ophthalmic ointment was applied on eye after injections. Mice were randomly assigned to study groups using a Random Number Table. Inoculation of the HSV-1 was performed 24 hours after the initial injection.

### Corneal Inoculation of TLR7 and TLR9 agonist

Following anesthetizing DPE-GFP×RAG1^{-/-} mice and application of topical proparacaine hydrochloride, central corneal epithelium was debrided using an Algerbrush II corneal rust ring remover with a 0.5-mm burr (Alger Equipment Co) 10 µg Imiquimod (TLR7 agonist; InvivoGen, San Diego, CA), 10 µg phosphorothioate CpG 1826 oligonucleotide (CpG-ODN; a synthetic TLR9 agonist; InvivoGen), or control oligonucleotide 1826 (Control ODN; InvivoGen) was topically administered on the eye. 24 hours later corneas were removed to sort corneal GFP-tagged pDCs and single cell PCR experiments.

### Local Adoptive Transfer of plasmacytoid Dendritic Cells

Following 24 hours of culture, 10⁴ isolated splenic pDCs were placed on the center of cornea of WT C57BL/6 mice using TISSEEL fibrin sealant (Baxter Healthcare Corporation), subsequent to debridement of corneal epithelium. Mice receiving tissue fibrin sealant only served as controls.

### Immunofluorescence Staining and Confocal Microscopy

Corneas were excised and were fixed in chilled acetone (Sigma-Aldrich), blocked in 2% bovine serum albumin (BSA; Sigma-Aldrich) and 1% anti-CD16/CD32 Fc receptor (FcR) mAb (2.4G2; Bio X Cell) for 30 minutes at RT, and incubated with combinations of fluorochrome-conjugated primary Abs including CD45, F4/80, Gr-1, or isotype controls (all BioLegend) overnight at 4°C. After washings, samples were mounted with DAPI-containing medium (Vector Laboratories Inc.), and imaged by confocal microscopy using a Leica TCS SP5 (Leica Microsystems, Wetzlar, Germany). Cell densities were quantified via IMARIS (Bitplane AG).

### Corneal Single Cell Suspension and Flow Cytometry

Normal WT C57BL/6 corneas (n=15-20) were pooled, cut into pieces, and digested via incubation with 2 mg/ml collagenase D (Roche, Indianapolis, IN) and 0.05 mg/ml DNAse (Roche) to yield single cells prior to flow cytometric analysis. Next, after blocking, samples were labeled with combinations of antibodies including CD45, PDCA-1, CD45R/B220, TGF-β1 or their respective isotype controls (all BioLegend). Samples were then washed and analyzed with a BD LSR II flow cytometer (BD Biosciences, San Jose, CA). Data were analysed with FlowJo V9.2 (FlowJo, LLC, Ashland, OR). Forward and side scatter plots were used to exclude dead cells, debris, and doublets. Experiments were repeated at least 3 times.

### Corneal and Splenic Plasmacytoid Dendritic Cell Sorting

Corneal GFP-tagged pDCs were sorted from pooled (n=10) collagenase-digested normal corneas of DPE-GFP×RAG-1^{-/-} mice. C57BL/6 mice were used as controls for GFP sorting. Splenic GFP⁺ pDCs were sorted from DPE-GFP×RAG-1^{-/-} mice for adoptive transfer experiments. To enhance pDC isolation yield, we injected 8-week old DPE-GFP×RAG1^{-/-} mice with 5 × 10⁶ B16 murine Flt3L-secreting melanoma tumor cells, as previously described (Bjorck, Blood 98(13):3520-3526, 2001; Brawand et al., J. Immunol. 169(12):6711-6719, 2002; Naik et al., Meth. Mol. Biol. 595:167-176, 2010). 10-14 days later, we harvested the spleens and sorted GFP⁺ pDCs. Briefly, spleens were harvested, mechanically dissociated and passed through a 40 µm cell strainer (BD Falcon) to yield single cell suspensions of splenic cells. Next, RBCs were lysed using ACK RBC lysis buffer (Biofluids). GFP⁺ pDCs were sorted using MoFlo Astrios EQ (Beckman Coulter).

### RNA Isolation, cDNA Synthesis, and Semi-Quantitative Real-time PCR

Corneal epithelium was removed with fine forceps following 30 minutes incubation with PBS containing 20 mM EDTA (Sigma-Aldrich) at 37°C. Next, 4-6 corneal stromas were pooled and lysed using BeadBug Microtube Homogenizer (Benchmark Scientific, Inc.). Next, RNA was isolated from the corneal stroma using RNeasy Plus Universal Mini kit (QIAGEN). RNA yield was measured by spectroscopy (NanoDrop ND-1000; NanoDrop Technologies, Inc.). cDNA was synthetized using 300 ng of template RNA using QuantiTect Reverse Transcription kit (Qiagen). For single cell PCR, RNA isolation and cDNA synthesis was performed via REPLI-g Cell WGA & WTA kit (Qiagen) on 100 GFP⁺ sorted corneal pDCs. qRT-PCR was performed using iTaq Universal SYBR Green Supermix (Biorad, Hercules, CA) and Bio-Rad CFX96 Real-Time PCR Detection System (Bio-rad, Hauppauge, NY) with the primers set forth in Table 2. Relative mRNA level was measured with ΔΔCT method.

### ELISA

Corneal epithelium was removed as above, corneal stormas were pooled (n=4-6), and homogenized in ice-cold RIPA lysis buffer containing 1 mM phenylmethylsulfonyl fluoride (PMSF; Sigma-Aldrich) and 30 µg/mL aprotinin (Sigma-Aldrich) at 4°C using Branson sonifier (Branson Ultrasonics, Danbury, CT). The homogenate was centrifuged at 15,000 g for 20 minutes at 4°C and the supernatant was analyzed using INF-α and TGF-β1 ELISA kits (both eBioscience).

### Statistical Analysis

Data was analyzed with SPSS version 17 (SPSS Inc., Chicago, IL). ANOVA with Scheffe host hoc was applied to assess differences among groups. p less than 0.05 was considered significant.

**Table 2. Primers**

| Transcript | Forward | Reverse |
|---|---|---|
| IFN-α | 5'-TCAATGACCTGCAAGGCTGTCTG-3' (SEQ ID NO: 17) | 5'-GGCATCTTCCTGGGTCAGGGGAAA-3' (SEQ ID NO: 18) |
| TGF-β1 | 5'-GGATACCAACTATTGCTTCAGCTCC-3' (SEQ ID NO: 19) | 5'-AGGCTCCAAATATAGGGGCAGGGTC-3' (SEQ ID NO: 20) |
| HSV-1 gB | 5'-AACGCGACGCACATCAAG-3' (SEQ ID NO: 21) | 5'-CTGGTACGCGATCAGAAAGC-3' (SEQ ID NO: 22) |
| GAPDH | 5'- CCCACTAACATCAAATGGGG-3' (SEQ ID NO: 23) | 5'- GATGATGACCCTTTTGGCTC-3' (SEQ ID NO: 24) |

### Example 5: Corneal pDCs Modulate Sterile Corneal Inflammation

### Results

In order to study the effect of corneal pDCs in non-infectious inflammation, we used the mouse model of corneal sterile inflammation by intrastromal suture placement. Similar to experiments described above, we depleted corneal pDCs by injecting 30 ng DT subconjunctivally to pDC-DTR mice. Control groups consisted of WT C57BL/6 mice receiving subconjuctival DT and pDC-DTR mice treated with subconjunctival PBS (referred to as sham-depleted in this section). We repeated the injections every other day to prevent repopulation of the pDCs. One day after initial injection, we induced corneal inflammation by suture placement. We observed an increased opacity at day 7 and 14 after suture placement in those corneas ablated of pDCs (Fig. 22A). Also, we observed increased density of innate immune cells at day 7 following suture placement evident by the increase in the density of CD45⁺ leukocytes (1.8 fold), Gr-1⁺ neutrophils (3 fold), and F4/80⁺ macrophages (3 fold; Fig. 22B). These findings suggest that pDCs have anti-inflammatory functions.

### Experimental Methods

### Animals

Six- to ten-week-old male wild-type (WT) C57BL/6 mice were purchased from Charles River (Charles River Laboratories International, Wilmington, MA); BDCA2-DTR mice (C57BL/6 background; called pDC-DTR; Jackson Laboratory, Bar Harbor, ME) (Swiecki et al., Immunity 33(6):955-966, 2010) were bred in house in specific pathogen free conditions. pDC-DTR mouse were bred to homozygousity for the experiments. All protocols were approved by Schepens Eye Research Institute, and Tufts Medical Center and Tufts University School of Medicine Animal Care and Use Committees (IACUC), and animals were treated according to the Association for Research in Vision and Ophthalmology (ARVO) Statement for the Use of Animals in Ophthalmic and Vision Research.

### Corneal Suture Placement

Under deep anesthesia and following application of topical proparacaine hydrochloride, corneal suture placement was performed on WT C57BL/6 and pDC-DTR mice as previously described (Cursiefen et al., Proc. Natl. Acad. Sci. U.S.A. 103(30):11405-11410, 2006; Streilein et al., Invest. Ophthal. Vis. Sci. 37(2):413-424, 1996). Briefly, three 11-0 nylon sutures (Sharpoint; Vanguard, Houston, TX) were placed through the paracentral stroma of the mice, each 120° apart, without perforating the cornea, using aseptic microsurgical technique and an operating microscope.

### Clinical Evaluation of Corneal Opacity

Corneal opacities were scored using the following scoring: 0, normal; 1, corneal opacity confined to less than one quarter of the cornea with visible iris; 2, corneal opacity between one quarter and one half of the cornea with visible iris; 3, corneal opacity extended to greater than half of the cornea with partially invisible iris; and 4, maximal corneal opacity spread over the entire cornea and completely invisible iris.

### Immunofluorescence Staining and Confocal Microscopy

7 and 14 days following suture placement, corneas were harvested, fixed in chilled acetone (Sigma-Aldrich), blocked in 2% bovine serum albumin (BSA; Sigma-Aldrich) and 1% anti-CD16/CD32 Fc receptor (FcR) mAb (2.4G2; Bio X Cell) for 30 minutes at RT, and incubated with combinations of fluorochrome-conjugated primary Abs including CD45, Gr-1, and F4/80 (all BioLegend) overnight at 4°C. After washings, samples underwent confocal microscopy. Cell densities were quantified via IMARIS (Bitplane AG).

### Statistical Analysis

Data was analyzed with SPSS version 17 (SPSS Inc., Chicago, IL). ANOVA with Scheffe host hoc was applied to assess differences among groups. p less than 0.05 was considered significant.

Use of singular forms herein, such as "a" and "the," does not exclude indication of the corresponding plural form, unless the context indicates to the contrary. Similarly, use of plural terms does not exclude indication of a corresponding singular form.

## Claims

1. Plasmacytoid dendritic cell (pDC) or composition comprising a plasmacytoid dendritic cell and a pharmaceutically acceptable carrier or diluent for use in preventing or treating a disease or condition of the eye in a subject, wherein said pDC or composition is administered to an eye of the subject by intravitreal or sub-retinal injection or by topical administration using a tissue adhesive.

2. Plasmacytoid dendritic cell (pDC) or composition comprising a plasmacytoid dendritic cell and a pharmaceutically acceptable carrier or diluent for use according to claim 1, wherein the disease or condition of the eye is **characterized by** neovascularization, such as corneal, retinal or choroidal neovascularization.

3. Plasmacytoid dendritic cell (pDC) or composition comprising a plasmacytoid dendritic cell and a pharmaceutically acceptable carrier or diluent for use according to claim 2, wherein the neovascularization is corneal neovascularization and the subject has or is at risk of developing corneal infection, inflammation, autoimmune disease, limbal stem cell deficiency, neoplasia, uveitis, keratitis, corneal ulcers, glaucoma, rosacea, lupus, dry eye disease, or ocular damage due to trauma, surgery, or contact lens wear.

4. Plasmacytoid dendritic cell (pDC) or composition comprising a plasmacytoid dendritic cell and a pharmaceutically acceptable carrier or diluent for use according to claim 2, wherein the neovascularization is retinal neovascularization and the subject has or is at risk of developing ischemic retinopathy, diabetic retinopathy, retinopathy of prematurity, retinal vein occlusion, ocular ischemic syndrome, sickle cell disease, Eales' disease, or macular degeneration.

5. Plasmacytoid dendritic cell (pDC) or composition comprising a plasmacytoid dendritic cell and a pharmaceutically acceptable carrier or diluent for use according to claim 2, wherein the neovascularization is choroidal neovascularization and the subject has or is at risk of developing inflammatory neovascularization with uveitis, macular degeneration, ocular trauma, sickle cell disease, pseudoxanthoma elasticum, angioid streaks, optic disc drusen, myopia, malignant myopic degeneration, or histoplasmosis.

6. Plasmacytoid dendritic cell (pDC) or composition comprising a plasmacytoid dendritic cell and a pharmaceutically acceptable carrier or diluent for use according to claim 1, wherein the disease or condition of the eye is **characterized by** ocular nerve degeneration or damage, such as corneal nerve damage.

7. Plasmacytoid dendritic cell (pDC) or composition comprising a plasmacytoid dendritic cell and a pharmaceutically acceptable carrier or diluent for use according to claim 6, wherein the subject has or is at risk of developing dry eye disease, corneal infection, or corneal neurotrophic keratopathy.

8. Plasmacytoid dendritic cell (pDC) or composition comprising a plasmacytoid dendritic cell and a pharmaceutically acceptable carrier or diluent for use according to claim 6, wherein the subject has or is at risk of experiencing ocular damage due to trauma, surgery, contact lens wear, dry eye disease, herpetic keratitis that is optionally caused by HSV-1, neurotrophic keratitis, corneal infections, excessive or improper contact lens wear, ocular herpes (HSV), herpes zoster (shingles), chemical and physical burns, injury, trauma, surgery (including corneal transplantation, laser assisted in-situ keratomileusis (LASIK), penetrating keratoplasty (PK), automated lamellar keratoplasty (ALK), photorefractive keratectomy (PRK), radial keratotomy (RK), cataract surgery, and corneal incisions), abuse of topical anesthetics, topical drug toxicity, corneal dystrophies, vitamin A deficiency, diabetes, and microbial keratitis.

9. Plasmacytoid dendritic cell (pDC) or composition comprising a plasmacytoid dendritic cell and a pharmaceutically acceptable carrier or diluent for use according to any of the previous claims, wherein the plasmacytoid dendritic cell is applied to the cornea of the subject.

10. Plasmacytoid dendritic cell (pDC) or composition comprising a plasmacytoid dendritic cell and a pharmaceutically acceptable carrier or diluent for use according to any of the claims 1 to 8, wherein the plasmacytoid dendritic cell is administered to the subject by intravitreal or sub-retinal injection.

11. Plasmacytoid dendritic cell (pDC) or composition comprising a plasmacytoid dendritic cell and a pharmaceutically acceptable carrier or diluent for use according to any of the previous claims, wherein the plasmacytoid dendritic cell is obtained from the subject to whom it is administered or wherein the plasmacytoid dendritic cell is obtained from an individual and/or species different from the subject to whom it is administered.

## Patentansprüche

1. Plasmazytoide dentritische Zelle (pDC) oder Zusammensetzung, die eine plasmazytoide dentritische Zelle und einen pharmazeutisch verträglichen Träger oder Verdünner umfasst, zur Verwendung bei der Prävention oder Behandlung einer Erkrankung oder eines Leidens des Auges eines Subjekts, wobei die pDC oder die Zusammensetzung durch intravitreale oder sub-retinale Injektion oder durch topische Verabreichung mit Hilfe eines Gewebeklebers an ein Auge des Subjekts verabreicht wird.

2. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 1, wobei die Erkrankung oder das Leiden des Auges durch Neovaskularisation, wie beispielsweise korneale, retinale oder choroidale Neovaskularisation gekennzeichnet ist.

3. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 2, wobei die Neovaskularisation eine korneale Neovaskularisation ist und das Subjekt eine korneale Infektion, eine Entzündung, eine Autoimmunerkrankung, eine limbale Stammzelleninsuffizienz, eine Neoplasie, eine Uveitis, eine Keratitis, korneale Geschwüre, ein Glaukom, eine Rosazea, einen Lupus, Trockenes Auge oder Augenschäden durch Trauma, Operation oder das Tragen von Kontaktlinsen hat oder ein Risiko, diese zu entwickeln, hat.

4. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 2, wobei die Neovaskularisation eine retinale Neovaskularisation ist und das Subjekt eine ischämische Retinopathie, diabetische Retinopathie, Frühgeborenen-Retinopathie, einen Netzhautvenenverschluss, ein ischämisches Augensyndrom, eine Sichelzellenanämie, eine Eales-Krankheit oder eine Makuladegeneration hat oder ein Risiko, diese zu entwickeln, hat.

5. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 2, wobei die Neovaskularisation eine choroidale Neovaskularisation ist und das Subjekt eine entzündliche Neovaskularisation mit Uveitis, eine Makuladegeneration, ein Augentrauma, eine Sichelzellenanämie, ein Pseudoxanthoma elasticum, Angioid streaks, Drusenpapillen, Myopie, maligne degenerative Myopie oder Histoplasmose hat oder ein Risiko, diese zu entwickeln, hat.

6. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 1, wobei die Erkrankung oder das Leiden des Auges durch Augennervendegeneration oder -schäden, wie beispielsweise Schäden der kornealen Nerven, gekennzeichnet ist.

7. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 6, wobei das Subjekt Trockenes Auge, eine korneale Infektion oder eine korneale neurotrophe Keratopathie hat oder ein Risiko, diese zu entwickeln, hat.

8. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach Anspruch 6, wobei das Subjekt Augenschäden durch Trauma, Operation oder das Tragen von Kontaktlinsen, Trockenes Auge, Herpes-Keratitis, die optional durch HSV-1 verursacht ist, neurotrophe Keratitis, korneale Infektionen, übermäßiges oder unsachgemäßes Tragen von Kontaktlinsen, Augenherpes (HSV), Herpes zoster (Gürtelrose), chemische und physikalische Verbrennungen, Verletzungen, Trauma, Operation (einschließlich Kornealtransplantation, Laser-in-situ-Keratomileusis (LASIK), perforierender Keratoplastik (PK), automatischer lamelläre Keratoplastik (ALK), photorefraktiver Keratektomie (PRK), radiäre Keratotomie (RK), Kataraktchirurgie, und kornealer Inzisionen), Missbrauch topischer Anästhetika, topische Arzneimitteltoxizität, korneale Dystrophien, Vitamin-A-Mangel, Diabetes und mikrobielle Keratitis hat oder ein Risiko, diese zu erleiden, hat.

9. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach einem der vorhergehenden Ansprüche, wobei die plasmazytoide dentritische Zelle auf die Kornea des Subjekts angewandt wird.

10. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach einem der Ansprüche 1 bis 8, wobei die plasmazytoide dentritische Zelle durch intravitreale oder sub-retinale Injektion an das Subjekt verabreicht wird.

11. Plasmazytoide dentritische Zelle (pDC) oder Zusammensetzung, die eine plasmazytoide dentritische Zelle und einen pharmazeutisch verträglichen Träger oder Verdünner umfasst, zur Verwendung nach einem der vorhergehenden Ansprüche, wobei die plasmazytoide dentritische Zelle von dem Subjekt gewonnen wird, an das sie verabreicht wird, oder wobei die plasmazytoide dentritische Zelle von einem Individuum und/oder einer Spezies gewonnen wird, das/die sich von dem Subjekt unterscheidet, an das sie verabreicht wird.

## Revendications

1. Cellule dendritique plasmacytoïde (pDC) ou composition comprenant une cellule dendritique plasmacytoïde et un vecteur ou un diluant pharmaceutiquement acceptable pour une utilisation dans la prévention ou le traitement d'une maladie ou d'une affection de l'oeil chez un sujet, dans laquelle ladite pDC ou composition est administrée à un oeil du sujet par injection intravitréenne ou sous-rétinienne ou par administration topique en utilisant un adhésif tissulaire.

2. Cellule dendritique plasmacytoïde (pDC) ou composition comprenant une cellule dendritique plasmacytoïde et un vecteur ou un diluant pharmaceutiquement acceptable pour une utilisation selon la revendication 1, dans laquelle la maladie ou l'affection de l'oeil est **caractérisée par** une néovascularisation, telle qu'une néovascularisation cornéenne, rétinienne ou choroïdienne.

3. Cellule dendritique plasmacytoïde (pDC) ou composition comprenant une cellule dendritique plasmacytoïde et un vecteur ou un diluant pharmaceutiquement acceptable pour une utilisation selon la revendication 2, dans laquelle la néovascularisation est une néovascularisation cornéenne et le sujet présente ou présente un risque de développer une infection cornéenne, une inflammation, une maladie autoimmune, une déficience en cellules souches limbiques, une néoplasie, une uvéite, une kératite, des ulcères cornéens, un glaucome, une rosacée, un lupus, une maladie de l'oeil sec, ou des lésions oculaires dues à un traumatisme, à une chirurgie, ou au port de lentilles de contact.

4. Cellule dendritique plasmacytoïde (pDC) ou composition comprenant une cellule dendritique plasmacytoïde et un vecteur ou un diluant pharmaceutiquement acceptable pour une utilisation selon la revendication 2, dans laquelle la néovascularisation est une néovascularisation rétinienne et le sujet présente ou présente un risque de développer une rétinopathie ischémique, une rétinopathie diabétique, une rétinopathie de prématurité, une occlusion veineuse rétinienne, un syndrome ischémique oculaire, une drépanocytose, une maladie d'Eales ou une dégénérescence maculaire.

5. Cellule dendritique plasmacytoïde (pDC) ou composition comprenant une cellule dendritique plasmacytoïde et un vecteur ou un diluant pharmaceutiquement acceptable pour une utilisation selon la revendication 2, dans laquelle la néovascularisation est une néovascularisation choroïdienne et le sujet présente ou présente un risque de développer une néovascularisation inflammatoire avec une uvéite, une dégénérescence maculaire, un traumatisme oculaire, une drépanocytose, un pseudoxanthome élastique, des stries angioïdes, des drusen de disque optique, une myopie, une myopie dégénérative maligne, ou une histoplasmose.

6. Cellule dendritique plasmacytoïde (pDC) ou composition comprenant une cellule dendritique plasmacytoïde et un vecteur ou un diluant pharmaceutiquement acceptable pour une utilisation selon la revendication 1, dans laquelle la maladie ou l'affection de l'oeil est **caractérisée par** une dégénérescence ou une lésion du nerf oculaire, telle qu'une lésion du nerf cornéen.

7. Cellule dendritique plasmacytoïde (pDC) ou composition comprenant une cellule dendritique plasmacytoïde et un vecteur ou un diluant pharmaceutiquement acceptable pour une utilisation selon la revendication 6, dans laquelle le sujet présente ou présente un risque de développer une maladie de l'oeil sec, une infection cornéenne, ou une kératopathie neurotrophique cornéenne.

8. Cellule dendritique plasmacytoïde (pDC) ou composition comprenant une cellule dendritique plasmacytoïde et un vecteur ou un diluant pharmaceutiquement acceptable pour une utilisation selon la revendication 6, dans laquelle le sujet présente ou présente un risque d'être atteint de lésions oculaires dues à un traumatisme, à une chirurgie, au port de lentilles de contact, à une maladie de l'oeil sec, à une kératite herpétique qui est facultativement provoquée par le HSV-1, à une kératite neurotrophique, à des infections cornéennes, au port excessif ou incorrect de lentilles de contact, à un herpès oculaire (HSV), à un zona, à des brûlures chimiques et physiques, à des blessures, à un traumatisme, à une chirurgie (y compris transplantation cornéenne, kératomileusis in situ assisté par laser (LASIK), kératoplastie perforante (PK), kératoplastie lamellaire automatisée (ALK), kératectomie photoréfractive (PRK), kératotomie radiale (RK), chirurgie de la cataracte, et incisions cornéennes), à un abus d'anesthésiques topiques, à une toxicité médicamenteuse topique, à des dystrophies cornéennes, à une déficience en vitamine A, à un diabète et à une kératite microbienne.

9. Cellule dendritique plasmacytoïde (pDC) ou composition comprenant une cellule dendritique plasmacytoïde et un vecteur ou un diluant pharmaceutiquement acceptable pour une utilisation selon l'une quelconque des revendications précédentes, dans laquelle la cellule dendritique plasmacytoïde est appliquée sur la cornée du sujet.

10. Cellule dendritique plasmacytoïde (pDC) ou composition comprenant une cellule dendritique plasmacytoïde et un vecteur ou un diluant pharmaceutiquement acceptable pour une utilisation selon l'une quelconque des revendications 1 à 8, dans laquelle la cellule dendritique plasmacytoïde est administrée au sujet par injection intravitréenne ou sous-rétinienne.

11. Cellule dendritique plasmacytoïde (pDC) ou composition comprenant une cellule dendritique plasmacytoïde et un vecteur ou un diluant pharmaceutiquement acceptable pour une utilisation selon l'une quelconque des revendications précédentes, dans laquelle la cellule dendritique plasmacytoïde est obtenue auprès du sujet auquel elle est administrée ou dans laquelle la cellule dendritique plasmacytoïde est obtenue auprès d'un individu et/ou d'une espèce différent(e) du sujet auquel elle est administrée.
